# EUROPEAN PATENT APPLICATION

(11) **EP 4 151 659 A1**
(43) Date of publication of application: **22.03.2023**
(21) Application number: 21804660.5
(22) Date of filing: 10.05.2021
(51) Int. Cl.: C07K 19/00, C12N 15/62

(54) **BIOLOGICAL MACROMOLECULAR TARGET-SPECIFIC COMPLEMENT INHIBITOR, PREPARATION METHOD THEREFOR, AND APPLICATION THEREOF**

(30) Priority: 11.05.2020 CN 202010393721
(71) Applicant: Shanghai Comgen Biopharmaceutical Co., Ltd., Shanghai 201315 (CN)
(72) Inventor: LI, Luying, Shanghai 201315 (CN)
(74) Representative: Böhm, Brigitte
(86) International application number: PCT/CN2021/092861
(87) International publication number: WO 2021/228052

(57) **Abstract**

A fusion protein of a biological macromolecular target-specific complement inhibitor comprising: (i) a CRIg extracellular domain, (ii) a complement inhibiting domain, and (iii) a synergetic domain, a preparation method therefor and a use thereof, and a pharmaceutical composition comprising the fusion protein. The fusion protein not only has an obvious targeted complement inhibiting effect, but also facilitates improving druggability and/or production, and thus can be used for treating and preventing a plurality of human diseases related to abnormal complement activation.

## Description

This application is a National Stage Application and claims priority under 35 U.S.C. § 371 to Patent Cooperation Treaty application PCT/CN2021/092861, filed May 10, 2021, which claims the benefit of CN application 202010393721.9, filed May 11, 2020. Priority is claimed to these applications and the disclosures of these prior applications are considered part of the disclosure of this application and to the extent allowed the entire contents of the aforementioned applications are incorporated herein.

### TECHNICAL FIELD

The present application relates to the field of biomedicine, and in particular, relates to the design, preparation and clinical application of a biomacromolecular targeted specific complement inhibitor.

### BACKGROUND ART

Complement system, as an important component of innate immunity and an important regulator of acquired immunity, acts as a vital bridge between the innate immunity and the adaptive immunity. The complement system mainly exists in blood circulation, and is a highly complex self-regulatable cascade reaction system consisting of more than 30 activating factors, inhibiting factors, and complement receptors. Its main physiological function is to remove invading pathogenic bacteria and microorganisms, as well as host cell debris, and to coordinate the entire immune and inflammatory process. It is a key system (Ricklin et al., 2010) for immune surveillance and self-stabilization.

Generally, the complement system can be activated by means of three pathways: a classical pathway, in which the complement system can be activated by IgG and IgM type antibodies; a lectin pathway, in which the complement system can be activated by mannose on a bacterial surface; and an alternative pathway, in which the complement system can be activated by a variety of components such as pathogen cell wall/membrane components or complement C3b analogs such as snake toxin, and can be additionally automatically activated by the hydration of C3.

After activation, the complement in turn exerts its physiological effects in three main ways (Dunkelberger and Song, 2010). First, C3a, and in particular C5a, which are produced by activating complement components C3 and C5, can respectively bind to their receptors C3aR or C5aR1 and C5L2 expressed on immune cells, to recruit a variety of immune cells and secrete proinflammatory cytokines (such as TNF-α, IL-1, and IL-6) and chemokines (such as MCP-1, MIP-2, KC, and CINC) (Riedemann et al., 2003), thereby producing a strong proinflammatory action, i.e. pro-inflammatory effect, locally at the site of complement activation. Second, another product of C3 activation, C3b, and its further degradation product, iC3b, can be inserted into the surface of complement-attacked target cells to "label" these foreign components, and to bind to a variety of receptors such as CR1/CR2/CR3/CR4/CRIg expressed on immune cells; and these foreign components are eventually phagocytosed or lysed by immune cells, i.e., the opsonization and phagocytosis effect. Third, another product of C5 activation, C5b, can also be inserted into the surface of complement-attacked target cells to bind to complements C6 and C7 to form a stable complex C5b-7, which further binds to C8 and C9; and C9 is eventually multimerized to form a complex C5b-9n, also known as the membrane attack complex (MAC), which eventually forms, in a target cell membrane, a pore with an inner diameter of 5 nm, an outer diameter of 20 nm, and a height of 15 nm, such that the osmotic pressure inside and outside the cell changes to directly lyse the cells (Tegla et al., 2011), i.e., the cell lysis effect.

To prevent the above physiological effects, resulting from complement activation, from "accidentally killing" normal body cells, the body has evolved to produce more than 10 types of complement control proteins, which are expressed on the cell membrane or circulate in the blood system to inhibit the complement activation at different stages of complement activation, for example, complement inhibitory proteins CR1, CD46, CD55 and CD59 expressed on the cell membrane, and C1-INH, C4BP, FH, Vitronectin, S protein, etc. circulating freely in the blood. It is these complement control proteins that enable body cells to avoid complement killing during complement activation.

The complement system is a self-protection immune mechanism of a human body under normal conditions, but under some abnormal conditions in the body, either elevated levels of complement activation products or decreased levels or even absence of complement control proteins can lead to excessive complement activation, resulting in the killing of autologous tissue cells and eventually the occurrence and development of diseases, including paroxysmal nocturnal hemoglobinuria (PNH), atypical aaemolytic uraemic syndrome (aHUS), generalized myasthenia gravis (gMG), neuromyelitis optica spectrum disorders (NMOSD), age-related macular degeneration (AMD), autoimmune hemolytic anemia, autoimmune thrombocytopenia, aplastic anemia, systemic lupus erythematosus, rheumatoid arthritis, ankylosing spondylitis, atherosclerosis, Parkinson's disease, Alzheimer's disease (senile dementia), asthma, allergies, psoriasis, multiple sclerosis, Crohn's disease, etc. (Ricklin and Lambris, 2007). The excessive complement activation is closely related to the occurrence and development of autoimmune diseases, in particular in the early pathogenic stage. Therefore, inhibitors against the complement system can intervene early in the progression of the aforementioned diseases, showing great clinical advantages and demands.

In view of the importance of the complement system for the pathogenesis of numerous autoimmune diseases, acute and chronic infections or other diseases, almost 50 complement inhibitors from nearly 30 pharmaceutical companies have been developed in various stages, which indirectly indicates that there is still a need for the development of more desirable complement inhibitors.

### SUMMARY OF THE INVENTION

The present application provides a fusion protein, comprising: (i) a CRIg extracellular domain; (ii) a complement inhibitory domain comprising a protein selected from the group consisting of factor H (FH), CD55, CD46, CD59 and CR1, or a functional fragment thereof; and (iii) a synergetic domain comprising a protein selected from the group consisting of an IgG Fc domain and a human serum albumin, or a functional fragment thereof. The inventors of the present application unexpectedly found that CRIg linked to other complement control proteins FH, CD55, CD46, CD59 or CR1, and further linked to IgG Fc segments or HSA, which not only showed a more significant complement inhibitory effect, but also was more conducive to production, purification and increased druggability, for example, by improving pharmacokinetic (PK)/pharmacodynamic (PD) effects. In some cases, according to the present invention, CRIg capable of binding to a fragment C3b and/or iC3b (which is produced after the activation of a complement component C3) to produce a targeting action, and another component such as FH, CD55, CD46, CD59, or CR1 (all, except for CD59, of which simultaneously bind to different positions of C3b and/or iC3b more steadily compared with the single CRIg, FH, CD55, or CR1, thereby more effectively inhibiting the action of C3b/iC3b) having a complement inhibitory effect are further linked to an IgG Fc segment or HSA, whereby a fusion protein is prepared (for example, with a genetic engineering method), which delivers a recombinantly linked complement control protein to the site of local complement activation, resulting in targeted inhibition of complement activation. This type of drug is applicable to the treatment and prevention of a variety of human diseases associated with abnormal complement activation.

In one aspect, the present application provides a fusion protein, comprising: (i) a CRIg extracellular domain; (ii) a complement inhibitory domain comprising a protein selected from the group consisting of factor H (FH), CD55, CD46, CD59 and CR1, or a functional fragment thereof; and (iii) a synergetic domain comprising a protein selected from the group consisting of an IgG Fc domain and a human serum albumin, or a functional fragment thereof.

In some embodiments, said CRIg extracellular domain comprises an amino acid sequence as set forth in SEQ ID NO.: 6.

In some embodiments, a C-terminus of said CRIg extracellular domain is directly or indirectly linked to an N-terminus of said complement inhibitory domain.

In some embodiments, a C-terminus of said complement inhibitory domain is directly or indirectly linked to an N-terminus of said synergetic domain.

In some embodiments, an N-terminus of said CRIg extracellular domain is directly or indirectly linked to the C-terminus of said complement inhibitory domain.

In some embodiments, the C-terminus of said CRIg extracellular domain is directly or indirectly linked to the N-terminus of said synergetic domain.

In some embodiments, said indirect linking is performed via a linker.

In some embodiments, said linker comprises an amino acid sequence as set forth in any one of SEQ ID NOs.: 44, 46, 48, and 50.

In some embodiments, said complement inhibitory domain comprises an amino acid sequence as set forth in any one of SEQ ID NOs.: 8, 18, 20, 22, 62, and 64.

In some embodiments, said synergetic domain is a human serum albumin.

In some embodiments, said human serum albumin comprises an amino acid sequence as set forth in SEQ ID NO.: 12.

In some embodiments, said fusion protein has a single-chain structure.

In some embodiments, said fusion protein comprises said CRIg extracellular domain, said complement inhibitory domain, and said synergetic domain in sequence from an N-terminus to a C-terminus.

In some embodiments, said fusion protein comprises said complement inhibitory domain, said CRIg extracellular domain, and said synergetic domain in sequence from the N-terminus to the C-terminus.

In some embodiments, said fusion protein comprises an amino acid sequence as set forth in SEQ ID NO.: 14.

In some embodiments, said synergetic domain comprises an IgG Fc domain.

In some embodiments, said IgG comprises a protein selected from the group consisting of: human IgG1 and human IgG4.

In some embodiments, said IgG Fc domain comprises an amino acid sequence as set forth in any one of SEQ ID NOs.: 10, 30, 32, and 34.

In some embodiments, said fusion protein comprises a first polypeptide chain and a second polypeptide chain, wherein said first polypeptide chain comprises said CRIg extracellular domain, a first complement inhibitory domain, and a first said IgG Fc domain, and said second polypeptide chain comprises said CRIg extracellular domain, a second complement inhibitory domain, and a second IgG Fc structure domain, and wherein said first IgG Fc domain and said second IgG Fc domain are capable of interacting with each other to form a dimer.

In some embodiments, said first complement inhibitory domain and said second complement inhibitory domain each independently comprise a protein selected from the group consisting of factor H (FH), CD55, CD46, CD59 and CR1, or a functional fragment thereof.

In some embodiments, said first complement inhibitory domain is identical to said second complement inhibitory domain.

In some embodiments, said first IgG Fc domain is identical to said second IgG Fc domain.

In some embodiments, said first IgG Fc domain and said second IgG Fc domain each comprise an amino acid sequence as set forth in any one of SEQ ID NOs.: 10 and 30.

In some embodiments, said first polypeptide chain is identical to said second polypeptide chain.

In some embodiments, said first polypeptide chain and/or said second polypeptide chain comprise/comprises an amino acid sequence as set forth in any one of SEQ ID NOs.: 14, 24, 26, 28, 58, 60, 66, and 68.

In some embodiments, said first complement inhibitory domain is different from said second complement inhibitory domain.

In some embodiments, said first complement inhibitory domain and said second complement inhibitory domain each independently comprise a protein selected from the group consisting of CD59 and CD55, or a functional fragment thereof.

In some embodiments, said first complement inhibitory domain and said second complement inhibitory domain each independently comprise a protein selected from the group consisting of FH and CD55, or a functional fragment thereof.

In some embodiments, said first complement inhibitory domain and said second complement inhibitory domain each independently comprise a protein selected from the group consisting of CD46 and CD59, or a functional fragment thereof.

In some embodiments, said first IgG Fc domain is identical to said second IgG Fc domain.

In some embodiments, said first IgG Fc domain is different from said second IgG Fc domain.

In some embodiments, said first IgG Fc domain comprises an amino acid sequence as set forth in any one of SEQ ID NOs.: 32 and 34.

In some embodiments, said second IgG Fc domain comprises an amino acid sequence as set forth in any one of SEQ ID NOs.: 32 and 34.

In some embodiments, said first polypeptide chain comprises an amino acid sequence as set forth in any one of SEQ ID NOs: 36, 38, 40, and 42.

In some embodiments, said second polypeptide chain comprises an amino acid sequence as set forth in any one of SEQ ID NOs.: 36, 38, 40, and 42.

In some embodiments, in said fusion protein:
said first polypeptide chain comprises an amino acid sequence as set forth in SEQ ID NO.: 38, and said second polypeptide chain comprises an amino acid sequence as set forth in SEQ ID NO.: 40; or
said first polypeptide chain comprises an amino acid sequence as set forth in SEQ ID NO.: 36, and said second polypeptide chain comprises an amino acid sequence as set forth in SEQ ID NO.: 42;
said first polypeptide chain comprises an amino acid sequence as set forth in SEQ ID NO.: 38, and the second polypeptide chain comprises an amino acid sequence as set forth in SEQ ID NO.: 42.

In another aspect, the present application provides an isolated nucleic acid molecule or isolated nucleic acids, encoding said fusion protein or a fragment thereof.

In another aspect, the present application provides a vector comprising said nucleic acid molecule.

In another aspect, the present application provides a cell comprising said vector or expressing said fusion protein.

In another aspect, the present application provides a preparation method of the fusion protein as defined. The preparation method comprises the steps of: synthesizing said fusion protein, and/or, culturing said cell under a condition of allowing the expression of said fusion protein.

In another aspect, the present application provides a pharmaceutical composition comprising said fusion protein and an optionally pharmaceutically acceptable carrier.

In another aspect, the present application provides use of said fusion protein or said pharmaceutical composition in the preparation of a drug for treatment of diseases associated with targeted inhibition of complement activation.

In some embodiments, said diseases comprise autoimmune diseases.

In some embodiments, said diseases comprise autoimmune myasthenia gravis.

Other aspects and advantages of the present application can be readily perceived by those skilled in the art from the detailed description below. The detailed description below only shows and describes the exemplary embodiments of the present application. As would be appreciated by those skilled in the art, the content of the present application allows those killed in the art to change the specific embodiments disclosed without departing from the spirit and scope involved in the present application. Accordingly, the accompanying drawings and the description in the specification of the present application are merely for an exemplary but not restrictive purpose.

### BRIEF DESCRIPTION OF THE DRAWINGS

The specific features of the present invention involved in the present application are listed in the appended claims. The characteristics and advantages of the present invention involved in the present application can be better understood by referring to the exemplary embodiments and the accompanying drawings described in detail below. A brief description of the drawings is as follows:
FIG. 1 shows the schematic design diagrams of (CRIg-FH-IgG4Fc)×2 and CRIg-FH-HSA;
FIG. 2 shows the identification of (CRIg-FH-IgG4Fc)×2 and CRIg-FH-HSA recombinant proteins;
FIG. 3 shows the inhibitory effect of (CRIg-FH-IgG4Fc)×2 against the classical pathway of human serum complements;
FIG. 4 shows the inhibitory effect of (CRIg-FH-IgG4Fc)×2 against the alternative pathway of human serum complements;
FIG. 5 shows the inhibitory effect of CRIg-FH-HSA against the classical pathway of human serum complements;
FIG. 6 shows the inhibitory effect of CRIg-FH-HSA against the alternative pathway of human serum complements;
FIG. 7 shows the pharmacokinetic assay of (CRIg-FH-IgG4Fc)×2 in rats;
FIG. 8 shows the pharmacokinetic assay of CRIg-FH-HSA in rats;
FIG. 9 shows the schematic design diagrams of (CRIg-FH-IgG4Fc)×2, (CRIg-CD55-IgG4Fc)×2, (CRIg-CD46-IgG4Fc)×2, and (CRIg-CD59-IgG4Fc)×2;
FIG. 10 shows the identification of four recombinant proteins, including (CRIg-FH-IgG4Fc)×2, (CRIg-CD55-IgG4Fc)×2, (CRIg-CD46-IgG4Fc)×2, and (CRIg-CD59-IgG4Fc)×2;
FIG. 11 shows the inhibitory effect of (CRIg-CD55-IgG4Fc)×2 against the classical pathway of human serum complements;
FIG. 12 shows the inhibitory effect of (CRIg-CD55-IgG4Fc)×2 against the alternative pathway of human serum complements;
FIG. 13 shows the inhibitory effect of (CRIg-CD46-IgG4Fc)×2 against the classical pathway of human serum complements;
FIG. 14 shows the inhibitory effect of (CRIg-CD46-IgG4Fc)×2 against the alternative pathway of human serum complements;
FIG. 15 shows the inhibitory effect of (CRIg-CD59-IgG4Fc)×2 against the classical pathway of human serum complements;
FIG. 16 shows the inhibitory effect of (CRIg-CD59-IgG4Fc)×2 against the alternative pathway of human serum complements;
FIG. 17 shows the schematic design diagrams of three recombinant proteins, including (CRIg-CD55-IgG1 Fc) (CRIg-FH-IgG1 Fc), (CRIg-CD46-IgG1 Fc) (CRIg-CD59-IgG1 Fc), and (CRIg-CD55-IgG1 Fc) (CRIg-CD59-IgG1 Fc);
FIG. 18 shows the identification of three recombinant proteins, including (CRIg-CD55- IgG 1 Fc) (CRIg-FH-IgG1 Fc), (CRIg-CD46-IgG1 Fc) (CRIg-CD59-IgG1 Fc), and (CRIg-CD55-IgG1 Fc) (CRIg-CD59-IgG1 Fc);
FIG. 19 shows the inhibitory effect of (CRIg-CD55-IgG1 Fc) (CRIg-FH-IgG1 Fc) against the classical pathway of human serum complements;
FIG. 20 shows the inhibitory effect of (CRIg-CD55-IgG1 Fc) (CRIg-FH-IgG1 Fc) against the alternative pathway of human serum complements;
FIG. 21 shows the inhibitory effect of (CRIg-CD46-IgG1 Fc) (CRIg-CD59-IgG1 Fc) against the classical pathway of human serum complements;
FIG. 22 shows the inhibitory effect of (CRIg-CD46-IgG1 Fc) (CRIg-CD59-IgG1 Fc) against the alternative pathway of human serum complements;
FIG. 23 shows the inhibitory effect of (CRIg-CD55-IgG1 Fc) (CRIg-CD59-IgG1 Fc) against the classical pathway of human serum complements;
FIG. 24 shows the inhibitory effect of (CRIg-CD55-IgG1 Fc) (CRIg-CD59-IgG1 Fc) against the alternative pathway of human serum complements;
FIG. 25 shows the changes in body weight of EAMG rats treated with a (CRIg-CD59-IgG4Fc)×2 drug;
FIG. 26 shows the changes in clinical score of EAMG rats treated with a (CRIg-CD59-IgG4Fc)×2 drug;
FIG. 27 shows the changes in death rate of EAMG rats treated with a (CRIg-CD59-IgG4Fc)×2 drug;
FIG. 28 shows the results of pharmacokinetic assay of CRIg-FH in rats;
FIG. 29 shows the schematic design diagrams of five recombinant proteins, including (CRIg-IgG4 Fc)×2, (FH-IgG4 Fc)×2, (FH-CRIg-IgG4Fc)×2, (CRIg-FH-IgG4Fc)×2, and (CRIg-L-FH-IgG4Fc)×2;
FIG. 30 shows the identification of five recombinant proteins, including (CRIg-IgG4 Fc)×2, (FH-IgG4 Fc)×2, (FH-CRIg-IgG4Fc)×2, (CRIg-FH-IgG4Fc)×2, and (CRIg-L-FH-IgG4Fc)×2;
FIG. 31 shows the inhibitory effects of five recombinant proteins, including (CRIg-IgG4 Fc)×2, (FH-IgG4 Fc)×2, (FH-CRIg-IgG4Fc)×2, (CRIg-FH-IgG4Fc)×2, and (CRIg-L-FH-IgG4Fc)×2, against the alternative pathway of human serum complements;
FIG. 32 shows the schematic design diagram of (CRIg-CR1-IgG4Fc)×2;
FIG. 33 shows the identification of a (CRIg-CR1-IgG4Fc)×2 recombinant protein;
FIG. 34 shows the inhibitory effect of (CRIg-CR1-IgG4Fc)×2 against the classical pathway of human serum complements; and
FIG. 35 shows the inhibitory effect of (CRIg-CR1-IgG4Fc)×2 against the alternative pathway of human serum complements.

### DETAILED DESCRIPTION OF THE PREFERRED EMBODIMENTS

The embodiments of the invention of the present application will be illustrated by specific examples below. Those familiar with this technology can easily understand other advantages and effects of the invention of the present application from the content disclosed in the specification.

### TERMS & DEFINITIONS

In the present application, the term "complement inhibitory domain" generally refers to a substance capable of inhibiting the activation of a complement system. Generally, the complement system can be activated by means of three pathways: a classical pathway, in which the complement system can be activated by IgG and IgM type antibodies; a lectin pathway, in which the complement system can be activated by mannose on a bacterial surface; and an alternative pathway, in which the complement system can be activated by a variety of components such as pathogen cell wall/membrane components or complement C3b analogs such as snake toxin, and can be additionally automatically activated by the hydration of C3. The complement inhibitory domain can perform inhibition at different activation stages. The complement inhibitory domain of the present application can be derived from a complement control protein or a functional fragment thereof, encompassing complement inhibitors present in the blood circulation and complement membrane control proteins on the surfaces of cell membranes, comprising, but not limited to, C1-INH (C1 inhibitor), C4BP (C4 binding protein), factor I (FI), factor H (FH), S-protein, Clusterin, CD35 (also known as CR1), CD46 (also known as MCP), CD55 (also known as DAF) and/or CD59, and even the full-length or partial sequence of CRIg itself.

In the present application, the term "CD55" generally refers to a complement control protein, which also becomes a complement decay acceleration factor or DAF. The CD55 can regulate the complement system, and recognize C4b and C3b fragments produced during the activation of complement components C4 (via the classical or lectin pathway) or C3 (via the alternative pathway). The CD55 can interact with the cell-associated C4b in the classical and lectin pathways to interfere with the conversion of C2 to C2b, thereby preventing the formation of a C4b2b C3 convertase; and the CD55 can also interact with the C3b in the alternative pathway to interfere with the conversion of Bb by factor B, thereby preventing the formation of a C3bBb C3 convertase in the alternative pathway. The CD55 of the present application may comprise a full-length CD55 protein or a fragment thereof, as well as various variants (for example, mutants and isomers) thereof. For example, an exemplary nucleic acid molecule encoding the CD55 may comprise a nucleotide sequence as set forth in SEQ ID NO. 17, and an exemplary CD55 protein may comprise a protein sequence as set forth in SEQ ID NO. 18.

In the present application, the term "CD59" generally refers to a complement control protein, also known as a "membrane attack complex (MAC) inhibitory protein (MAC-IP)", "membrane inhibitor of reactive lysis (MIRL)", "membrane attack complex inhibitory factor (MACIF)" or protectin, which belongs to the LY6/uPAR/α-neurotoxin protein family. The CD59 can be linked to a host cell via a glycophosphatidylinositol (GPI) anchor. When complement activation leads to the deposition of a C5b678 complex on the host cell, the CD59 can prevent C9 polymerization and form a complement membrane attack complex. The CD59 of the present application may comprise a full-length CD59 protein or a fragment thereof, as well as various variants (for example, mutants and isomers) thereof. For example, an exemplary nucleic acid molecule encoding the CD59 may comprise a nucleotide sequence as set forth in SEQ ID NO. 21, and an exemplary CD59 protein may comprise a protein sequence as set forth in SEQ ID NO. 22.

In the present application, the term "CD46" generally refers to a complement control protein, also known as a membrane cofactor protein (MCP). Generally, the CD46 has a cofactor activity, and can protect a host cell from complement damage by inactivating (via lysis) complement components C3b and C4b by means of a serum factor I. The CD46 of the present application may comprise a full-length CD46 protein or a fragment thereof, as well as various variants thereof (for example, mutants and isomers). For example, an exemplary nucleic acid molecule encoding the CD46 may comprise a nucleotide sequence as set forth in SEQ ID NO. 19, and an exemplary CD59 protein may comprise a protein sequence as set forth in SEQ ID NO. 20.

In the present application, the term "factor H (FH)" generally refers to a complement control protein, which is a member of the complement activation family of regulatory factors. The FH has a major function of regulating the alternative pathway of the complement system to ensure that the complement system functions against pathogens or other hazardous substances without damaging host tissues. The sequence of FH typically has some highly conserved motifs, called short consensus repeats (SCRs). Each SCR consists of about 60 amino acids, with two disulfide bonds, one highly substituted high-variable loop, and 3-8 short SCR junctions of residues. The FH typically consists of 20 SCRs, and the functional characteristics of the FH are typically located on the SCRs. The FH of the present application may comprise a full-length FH protein or a fragment thereof (for example, one or more SCRs), as well as various variants (for example, mutants and isomers) thereof. For example, an exemplary nucleic acid molecule encoding the FH may comprise a nucleotide sequence as set forth in SEQ ID NO. 3, and an exemplary FH protein may comprise a protein sequence as set forth in SEQ ID NO. 4. For example, the FH of the present application may comprise SCR1-5 domains, the exemplary nucleic acid molecule encoding the SCR1-5 domains of the FH may comprise a nucleotide sequence as set forth in SEQ ID NO. 7, and the exemplary SCR1-5 domains of the FH may comprise a protein sequence as set forth in SEQ ID NO. 8. In some cases, the FH may comprise other SCR fragments.

In the present application, the term "CR1" generally refers to a complement control protein, also known as a C3b/C4b receptor or CD35. CR1 comprises a total of 30 SCR or complement control protein (CCP) sequences, with CCP1-3 binding to C4b, and CCP8-11 and CCP15-18 binding to C3b, thereby exerting a complement inhibitory effect. The CR1 of the present application may comprise CCP8-11 or CCP15-18, as well as various variants (for example, mutants and isomers) thereof. For example, the CR1 of the present application may comprise CCP8-11 domains, the exemplary nucleic acid molecule encoding the CCP8-11 domains of the CR1 protein may comprise a nucleotide sequence as set forth in SEQ ID NO. 61, and the exemplary CCP8-11 domains of the CR1 protein may comprise a protein sequence as set forth in SEQ ID NO. 62. For example, the CR1 of the present application may comprise CCP15-18 domains, the exemplary nucleic acid molecule encoding the CCP15-18 domains of the CR1 protein may comprise a nucleotide sequence as set forth in SEQ ID NO. 63, and the exemplary CCP15-18 domains of the CR1 protein may comprise a protein sequence as set forth in SEQ ID NO. 64. In some cases, the CR1 may comprise other CCP fragments. Since the CCP8-11 and CCP15-18 domains of the encoded CR1 differ by only 3 amino acids and are functionally identical, the CCP8-11 domains of the CR1 are studied as an example in the present application.

In the present application, the term "single-chain structure" generally refers to a chain of amino acids linked by covalent bonds (for example, peptide bonds). The single-chain structure can be produced by linking polypeptide fragments, or by linking nucleic acids encoding the polypeptide fragments before expression. For example, a plurality of polypeptide chains of the same or different origins may form a fusion protein having a single-chain structure.

In the present application, the term "human serum albumin (HSA)" generally refers to a globular protein encoded by a human gene ALB, and is typically found in plasma. A natural HSA consists of a single polypeptide chain comprising three domains, namely, Domain I, Domain II, and Domain III. Each domain may comprise two sub-regions A and B to form a cylindrical structure with opposing rabbets, thereby making the structure of HSA relatively flexible and hydrophilic. Herein, this term may comprise naturally originated (for example, plasma-derived) HSAs and artificially synthesized (for example, by the gene recombination technology) HSAs. This term encompasses full-length HSAs or functional fragments thereof. An exemplary nucleic acid molecule encoding the HSA may comprise a nucleotide sequence as set forth in SEQ ID NO. 11, and an exemplary HSA may comprise an amino acid sequence as set forth in SEQ ID NO. 12.

In the present application, the term "synergetic domain" generally refers to a polypeptide domain capable of enhancing a complement inhibitory effect. The complement inhibitory effect may comprise: enhancing the intensity of complement inhibitory activity of a substance having a complement inhibitory activity (for example, a complement inhibitory domain), increasing the duration of complement inhibitory action of a substance having a complement inhibitory activity (for example, a complement inhibitory domain), reducing the duration of degradation of a substance having a complement inhibitory activity (for example, a complement inhibitory domain), and/or extending the half-life of a drug containing a substance having a complement inhibitory activity (for example, a complement inhibitory domain). The synergetic domain may comprise a domain from HSA, IgG1, IgG2, IgG3 and/or IgG4 Fc. In the present application, the synergetic domain and the complement inhibitory domain are on the same polypeptide chain, or may be on different polypeptide chains.

In the present application, the term "IgG Fc domain" generally refers to an immunoglobulin Fc region or a domain thereof, which may comprise a Fc domain from IgG1, IgG2, IgG3 and/or IgG4.

In the present application, the term "CRIg" generally refers to a complement membrane control protein, which belongs to the immunoglobulin superfamily. The CRIg can produce an inhibitory effect early in a complement cascade reaction by specifically recognizing iC3b (inactivated C3b) and inhibiting the activation of a C3 convertase. The CRIg may comprise CRIgs of different origins of species, for example, human or mouse. In some cases, the CRIg may be derived from a human CRIg. The human CRIg may comprise a long-form CRIg, which comprises V-type and C2-type terminal Ig domains. The human CRIg may also comprise a short-form CRIg, which comprises a V-type terminal Ig domain. An exemplary nucleic acid sequence encoding the CRIg may be as set forth in SEQ ID NO.: 1, and an exemplary CRIg protein may comprise an amino acid sequence as set forth in SEQ ID NO.: 2. Generally, a functional region of the CRIg is located in its extracellular domain. The term "CRIg extracellular domain" generally encompasses an extracellular functional region of CRIg, and an exemplary nucleic acid sequence encoding the CRIg extracellular domain may be as set forth in SEQ ID NO.: 5, and an exemplary CRIg extracellular domain may comprise an amino acid sequence as set forth in SEQ ID NO.: 6.

In the present application, the term "vector" generally refers to a nucleic acid molecule capable of self-replication in a suitable host. It transfers an inserted nucleic acid molecule into and/or between host cells. The vector may include a vector mainly for inserting DNA or RNA into cells, a vector mainly for replicating DNA or RNA, and a vector mainly for expressing DNA or RNA transcription and/or translation. The vector also includes a carrier having a variety of the functions defined above. The vector may be a polynucleotide that may be transcribed and translated into a polypeptide when introduced into a suitable host cell. Generally, the vector may produce a desired expression product by culturing a suitable host cell containing the vector.

In the present application, the term "cell" generally refers to an individual cell, a cell line or a cell culture, which may contain or already contains a plasmid or vector comprising a nucleic acid molecule of the present application, or which is capable of expressing the antibody or antigen-binding fragment thereof in the present application. The cell may include a progeny of a single host cell. Due to natural, accidental or deliberate mutations, progeny cells and original parent cells may not be necessarily identical in terms of morphology or genome, as long as they are capable of expressing the antibody or antigen-binding fragment thereof in the present application. The cells can be obtained by transfecting cells *in vitro* using the vector of the present application. The cells may be prokaryotic cells (for example, *Escherichia coli*), or eukaryotic cells (for example, yeast cells, for example, COS cells, Chinese hamster ovary (CHO) cells, HeLa cells, HEK293 cells, COS-1 cells, NS0 cells, or myeloma cells). In some cases, the cells may be mammalian cells. For example, the mammalian cells may be CHO-K1 cells. In the present application, the term "recombinant cell" generally refers to a cell into which a recombinant expression vector is introduced. A recombinant host cell comprises not only certain specific cells, but also the progenies of these cells.

In the present application, the term "pharmaceutically acceptable carrier" generally comprises a pharmaceutically acceptable adjuvant, excipient, or stabilizer, which is nontoxic for the cells or mammals that are exposed to it the dose and concentration used. Generally, the physiologically acceptable carrier is a PH buffered aqueous solution. Examples of the physiologically acceptable carrier may comprise: buffers, such as phosphate, citrate, and other organic acids; antioxidants, including ascorbic acid; low-molecular-weight (less than about 10 residues) polypeptides, and proteins, such as serum albumin, gelatin, or immunoglobulin; hydrophilic polymers, such as polyvinylpyrrolidone; amino acids, such as glycine, glutamine, asparagine, arginine, or lysine; monosaccharides, disaccharides, and other carbohydrates, including glucose, mannose, or dextrin; chelating agents, such as EDTA; sugar alcohols, such as mannitol or sorbitol; salt-forming counterions, such as sodium; and/or nonionic surfactants, such as TWEEN^{™}, polyethylene glycol (PEG), and PLURONICS^{™}.

In the present application, the term "diseases associated with targeted inhibition of complement activation" generally refers to diseases caused by abnormal complement activation inhibition and excessive complement activation, and these diseases may be caused by the elevated levels and/or activities of complement activation products or the reduced levels and/or activities of complement control proteins (for example, CD55, CD59, and/or FH). For example, compared with normal conditions, the decreased expression of complement control proteins, the decreased levels of gene replication and/or transcription of complement control proteins, and the abnormal translation process of complement control proteins result in the inability to control complement activation. The excessive complement activation may cause abnormalities (for example, excess) of proinflammatory responses, opsonization and phagocytosis effects, and/or cell lysis effects. In some cases, the body produces autoantibodies against normal autoantigens; these autoantibodies bind to antigens to activate the complement pathway; and due to reduced levels and/or activities of substances associated with complement inhibition and activation (for example, complement control proteins), immune injuries are caused to autologous tissues, organs, and cells, eventually leading to autoimmune diseases. In some cases, the diseases associated with targeted inhibition of complement activation comprise autoimmune diseases. In some cases, the diseases associated with targeted inhibition of complement activation may be caused by acute or chronic infections. In some cases, the diseases associated with targeted inhibition of complement activation may be caused by Genetic mutation. The diseases associated with targeted inhibition of complement activation may be selected from: paroxysmal nocturnal hemoglobinuria (PNH), atypical aaemolytic uraemic syndrome (aHUS), generalized myasthenia gravis (gMG), neuromyelitis optica spectrum disorders (NMOSD), age-related macular degeneration (AMD), autoimmune hemolytic anemia, autoimmune thrombocytopenia, aplastic anemia, systemic lupus erythematosus, rheumatoid arthritis, ankylosing spondylitis, atherosclerosis, Parkinson's disease, Alzheimer's disease (senile dementia), asthma, allergies, psoriasis, multiple sclerosis, and Crohn's disease. For example, the diseases comprise autoimmune myasthenia gravis.

In the present application, the term "autoimmune myasthenia gravis" generally refers to a chronic autoimmune disease in which the signaling between nerves and muscles is blocked to compromise the strength of skeletal muscles. This disease may be caused by excessive complement activation, with an immune response attacking proteins, i.e., acetylcholine receptors or receptor-related proteins, in the postsynaptic membrane of a neuromuscular junction. The symptoms of autoimmune myasthenia gravis will gradually spread from the eye muscles to the face and neck muscles over time, inducing weakness, slurred speech, difficulty in chewing and swallowing, and/or breathing difficulty. Then, it gradually spreads from the head and neck to the rest of the body, eventually leading to generalized myasthenia gravis.

### DETAILED DESCRIPTION OF THE INVENTION

### Fusion Protein

In one aspect, the present application provides a fusion protein. The fusion protein may comprise (i) a CRIg extracellular domain. The CRIg extracellular domain of the present application may comprise an amino acid sequence as set forth in SEQ ID NO.: 6. In some cases, the CRIg extracellular domain may comprise an amino acid sequence that has at least 90% homology to an amino acid sequence as set forth in SEQ ID NO.: 6, for example, any one amino acid sequence that has at least 95%, at least 96%, at least 97%, at least 98%, or at least 99% homology to the amino acid sequence as set forth in SEQ ID NO.: 6.

In the present application, the fusion protein may comprise (ii) a complement inhibitory domain.

In some cases, the complement inhibitory domain may comprise a protein derived from FH and a functional fragment thereof, and the FH may comprise an amino acid sequence as set forth in SEQ ID NO.: 4. In some cases, the FH may comprise an amino acid sequence that has at least 90% homology to an amino acid sequence as set forth in SEQ ID NO.: 4, for example, any one amino acid sequence that has at least 95%, at least 96%, at least 97%, at least 98%, or at least 99% homology to the amino acid sequence as set forth in SEQ ID NO.: 4. In some embodiments, the complement inhibitory domain may comprise one or more SCR domains of FH. For example, the complement inhibitory domain may comprise the SCR1-5 domain of FH, and the SCR1-5 domain of FH may comprise an amino acid sequence as set forth in SEQ ID NO.: 8. In some cases, the FH may comprise an amino acid sequence that has at least 90% homology to an amino acid sequence as set forth in SEQ ID NO.: 8, for example, any one amino acid sequence that has at least 95%, at least 96%, at least 97%, at least 98%, or at least 99% homology to the amino acid sequence as set forth in SEQ ID NO.: 8.

In some cases, the complement inhibitory domain may comprise a protein derived from CD55 and a functional fragment thereof, and the CD55 may comprise an amino acid sequence as set forth in SEQ ID NO.: 18. In some cases, the CD55 may comprise an amino acid sequence that has at least 90% homology to an amino acid sequence as set forth in SEQ ID NO.: 18, for example, any one amino acid sequence that has at least 95%, at least 96%, at least 97%, at least 98%, or at least 99% homology to the amino acid sequence as set forth in SEQ ID NO.: 18.

In some embodiments, the complement inhibitory domain may comprise a protein derived from CD59 and a functional fragment thereof, and the CD59 may comprise an amino acid sequence as set forth in any one of SEQ ID NO.: 22. In some cases, the CD59 may comprise an amino acid sequence that has at least 90% homology to an amino acid sequence as set forth in SEQ ID NO.: 22, for example, any one amino acid sequence that has at least 95%, at least 96%, at least 97%, at least 98%, or at least 99% homology to the amino acid sequence as set forth in SEQ ID NO.: 22.

In some cases, the complement inhibitory domain may comprise a protein derived from CD46 or a functional fragment thereof, and the CD46 may comprise an amino acid sequence as set forth SEQ ID NO.: 20. In some cases, the CD46 may comprise an amino acid sequence that has at least 90% homology to an amino acid sequence as set forth in SEQ ID NO.: 20, for example, any one amino acid sequence that has at least 95%, at least 96%, at least 97%, at least 98%, or at least 99% homology to the amino acid sequence as set forth in SEQ ID NO.: 20.

In some cases, the complement inhibitory domain may comprise a protein derived from CR1 or a functional fragment thereof, and the CR1 may comprise an amino acid sequence as set forth SEQ ID NO.: 62. In some cases, the CR1 may comprise an amino acid sequence that has at least 90% homology to an amino acid sequence as set forth in SEQ ID NO.: 62, for example, any one amino acid sequence that has at least 95%, at least 96%, at least 97%, at least 98%, or at least 99% homology to the amino acid sequence as set forth in SEQ ID NO.: 62. Or, the CR1 may comprise an amino acid sequence as set forth in SEQ ID NO.: 64. In some cases, the CR1 may comprise an amino acid sequence that has at least 90% homology to an amino acid sequence as set forth in SEQ ID NO.: 64, for example, any one amino acid sequence that has at least 95%, at least 96%, at least 97%, at least 98%, or at least 99% homology to the amino acid sequence as set forth in SEQ ID NO.: 64.

The complement inhibitory domain of the present application may comprise a protein derived and selected from the group consisting of factor H (FH), CD55, CD46, CD59 and CR1, or a functional fragment thereof. In some cases, the complement inhibitory domain may comprise an amino acid sequence as set forth in any one of SEQ ID NOs.: 8, 18, 20, 22, 62, and 64. In some cases, the complement inhibitory domain may comprise an amino acid sequence that has at least 90% homology to an amino acid sequence as set forth in any one of SEQ ID NOs.: 8, 18, 20, 22, 62, and 64, for example, any one amino acid sequence that has at least 95%, at least 96%, at least 97%, at least 98%, or at least 99% homology to the amino acid sequence as set forth in any one of SEQ ID NOs.: 8, 18, 20, 22, 62, and 64.

The complement inhibitory domain of the present application may comprise a protein derived and selected from the group consisting of factor H (FH), CD55, CD59 and CR1, or a functional fragment thereof. In some cases, the complement inhibitory domain may comprise an amino acid sequence as set forth in any one of SEQ ID NOs.: 8, 18, 20, 22, 62, and 64. In some cases, the complement inhibitory domain may comprise an amino acid sequence that has at least 90% homology to an amino acid sequence as set forth in any one of SEQ ID NOs.: 8, 18, 20, 22, 62, and 64, for example, any one amino acid sequence that has at least 95%, at least 96%, at least 97%, at least 98%, or at least 99% homology to the amino acid sequence as set forth in any one of SEQ ID NOs.: 8, 18, 20, 22, 62, and 64.

In the present application, the fusion protein may comprise (iii) a synergetic domain.

In some cases, the synergetic domain may comprise an IgG Fc domain or a functional fragment thereof. In some cases, the IgG Fc domain may be an IgG Fc4 domain, which may comprise an amino acid sequence as set forth in SEQ ID NO.: 10. In some cases, the synergetic domain may comprise an amino acid sequence that has at least 90% homology to an amino acid sequence as set forth in SEQ ID NO.: 10, for example, any one amino acid sequence thathas at least 95%, at least 96%, at least 97%, at least 98%, or at least 99% homology to the amino acid sequence as set forth in SEQ ID NO.: 10.

In some cases, the IgG Fc domain may be an IgG Fc1 domain, which may comprise a mutant thereof. For example, the IgG Fc domain may mutate to obtain a desired spatial structure, for example, to form a knob-hole structure for better formation of a dimer. For example, the IgG Fc1 may comprise an amino acid sequence as set forth in any one of SEQ ID NOs.: 30, 32, and 34. In some cases, the synergetic domain may comprise an amino acid sequence that has at least 90% homology to an amino acid sequence as set forth in any one of SEQ ID NOs.: 30, 32, and 34, for example, any one amino acid sequence that has at least 95%, at least 96%, at least 97%, at least 98%, or at least 99% homology to the amino acid sequence as set forth in any one of SEQ ID NOs.: 30, 32, and 34.

In the present application, the synergetic domain may comprise a human serum albumin or a functional fragment thereof, and the human serum albumin may comprise an amino acid sequence as set forth in SEQ ID NO.: 12. In some cases, the synergetic domain may comprise an amino acid sequence that has at least 90% homology to an amino acid sequence as set forth in SEQ ID NO.: 12, for example, any one amino acid sequence that has at least 95%, at least 96%, at least 97%, at least 98%, or at least 99% homology to the amino acid sequence as set forth in SEQ ID NO.: 12.

In the present application, the fusion protein may comprise: (i) a CRIg extracellular domain, (ii) a complement inhibitory domain, and (iii) a synergetic domain. In the fusion protein, a C-terminus of the CRIg extracellular domain may be directly or indirectly linked to an N-terminus of the complement inhibitory domain, or, a C-terminus of the complement inhibitory domain may be directly or indirectly linked to an N-terminus of the synergetic domain. For example, the fusion protein may comprise the CRIg extracellular domain, the complement inhibitory domain, and the synergetic domain in sequence from an N-terminus to a C-terminus. For example, the fusion protein may comprise the complement inhibitory domain, the CRIg extracellular domain, and the synergetic domain in sequence from the N-terminus to the C-terminus.

In the present application, the indirect linking may comprise linking via a linker. For example, the linker may comprise a linker peptide.

In some cases, the C-terminus of the CRIg extracellular domain may be linked to the N-terminus of the complement inhibitory domain via a linker, and the linker may comprise an amino acid sequence as set forth in any one of SEQ ID NOs.: 44, 46, 48 and 50. In some cases, the nucleotide sequence encoding the linker may be as set forth in any one of SEQ ID NO.: 43, 45, 47, and 49. For example, the linker may be (Gly4Ser)₃.

In some cases, the C-terminus of the complement inhibitory domain may be linked to an N-terminus of the synergetic domain via a linker, and the linker may comprise an amino acid sequence as set forth in any one of SEQ ID NOs.: 44, 46, 48 and 50. In some cases, the nucleotide sequence encoding the linker may be as set forth in any one of SEQ ID NO.: 43, 45, 47, and 49. For example, the linker may be (Gly4Ser)₃.

In the present application, the fusion protein may comprise the CRIg extracellular domain, the linker, the complement inhibitory domain, and the synergetic domain in sequence from the N-terminus to the C-terminus.

In the present application, the fusion protein may comprise the complement inhibitory domain, the linkder, the CRIg extracellular domain, and the synergetic domain in sequence from the N-terminus to the C-terminus.

In the present application, the fusion protein may comprise the CRIg extracellular domain, the FH, and the IgG Fc domain (for example, IgG1 Fc or IgG4 Fc) in sequence from the N-terminus to the C-terminus. For example, the fusion protein may comprise an amino acid sequence as set forth in SEQ ID NO.: 14 or 40.

In the present application, the fusion protein may comprise the CRIg extracellular domain, the linker, the FH, and the IgG Fc domain (for example, IgG1 Fc or IgG4 Fc) in sequence from the N-terminus to the C-terminus. For example, the fusion protein may comprise an amino acid sequence as set forth in SEQ ID NO.: 60.

In the present application, the fusion protein may comprise the FH, the CRIg extracellular domain, and the IgG Fc domain (for example, IgG1 Fc or IgG4 Fc) in sequence from the N-terminus to the C-terminus. For example, the fusion protein may comprise an amino acid sequence as set forth in SEQ ID NO.: 58.

In the present application, the fusion protein may comprise the CRIg extracellular domain, the FH, and the HSA in sequence from the N-terminus to the C-terminus. For example, the fusion protein may comprise an amino acid sequence as set forth in SEQ ID NO.: 16.

In the present application, the fusion protein may comprise the CRIg extracellular domain, the CD59, and the IgG Fc domain (for example, IgG1 Fc or IgG4 Fc) in sequence from the N-terminus to the C-terminus. For example, the fusion protein may comprise an amino acid sequence as set forth in SEQ ID NO.: 28 or 42.

In the present application, the fusion protein may comprise the CRIg extracellular domain, the CD55, and the IgG Fc domain (for example, IgG1 Fc or IgG4 Fc) in sequence from the N-terminus to the C-terminus. For example, the fusion protein may comprise an amino acid sequence as set forth in SEQ ID NO.: 24 or 38.

In the present application, the fusion protein may comprise the CRIg extracellular domain, the CR1, and the IgG Fc domain (for example, IgG1 Fc or IgG4 Fc) in sequence from the N-terminus to the C-terminus. For example, the fusion protein may comprise an amino acid sequence as set forth in SEQ ID NO.: 66 or 68.

In the present application, the fusion protein may have a single-chain structure.

For example, the fusion protein may comprise the CRIg extracellular domain, the FH SCR1-5, and the human serum albumin in sequence from the N-terminus to the C-terminus. For example, the fusion protein may comprise an amino acid sequence as set forth in SEQ ID NO.: 16. For example, the fusion protein may comprise an amino acid that has at least 90% homology to an amino acid sequence as set forth in SEQ ID NO.: 16, for example, any one amino acid sequence that has at least 95%, at least 96%, at least 97%, at least 98%, or at least 99% homology to the amino acid sequence as set forth in SEQ ID NO.: 16.

In the present application, the fusion protein may comprise a first polypeptide chain and a second polypeptide chain.

In some cases, the first polypeptide chain may comprise the CRIg extracellular domain, a first complement inhibitory domain, and a first IgG Fc domain. In some cases, the first complement inhibitory domain may comprise a protein selected from the group consisting of factor H (FH), CD55, CD46, CD59 and CR1, or a functional fragment thereof. In some cases, the first complement inhibitory domain may comprise a protein selected from the group consisting of factor H (FH), CD55, CD46, CD59 and CR1, or a functional fragment thereof. In some cases, the first IgG Fc domain may comprise a protein selected from the group consisting of IgG1 Fc and IgG4 Fc, or a functional fragment thereof.

In some cases, the second polypeptide chain may comprise the CRIg extracellular domain, a second complement inhibitory domain, and a second IgG Fc domain. In some cases, the second complement inhibitory domain may comprise a protein selected from the group consisting of factor H (FH), CD55, CD46, CD59 and CR1, or a functional fragment thereof. In some cases, the second complement inhibitory domain may comprise a protein selected from the group consisting of factor H (FH), CD55, CD46, CD59 and CR1, or a functional fragment thereof. In some cases, the second IgG Fc domain may comprise a protein selected from the group consisting of IgG1 Fc and IgG4 Fc, or a functional fragment thereof.

In some cases, the first polypeptide chain may comprise an amino acid sequence as set forth in any one of SEQ ID NOs.: 14, 24, 26, 28, 38, 40, 42, 58, 60, 66, and 68. For example, the first polypeptide chain may comprise an amino acid sequence that has at least 90% homology to an amino acid sequence as set forth in any one of SEQ ID NOs.: 14, 24, 26, 28, 38, 40, 42, 58, 60, 66, and 68, for example, any one amino acid sequence that has at least 95%, at least 96%, at least 97%, at least 98%, or at least 99% homology to the amino acid sequence as set forth in any one of SEQ ID NOs.: 14, 24, 26, 28, 38, 40, 42, 58, 60, 66, and 68.

In some cases, the first polypeptide chain may comprise an amino acid sequence as set forth in any one of SEQ ID NOs.: 14, 24, 28, 38, 40, 42, 58, 60, 66, and 68. For example, the first polypeptide chain may comprise an amino acid sequence that has at least 90% homology to an amino acid sequence as set forth in any one of SEQ ID NOs.: 14, 24, 28, 38, 40, 42, 58, 60, 66, and 68, for example, any one amino acid sequence that has at least 95%, at least 96%, at least 97%, at least 98%, or at least 99% homology to the amino acid sequence as set forth in any one of SEQ ID NOs.: 14, 24, 28, 38, 40, 42, 58, 60, 66, and 68.

In some cases, the second polypeptide chain may comprise an amino acid sequence as set forth in any one of SEQ ID NOs.: 14, 24, 26, 28, 38, 40, 42, 58, 60, 66, and 68. For example, the second polypeptide chain may comprise an amino acid sequence that has at least 90% homology to an amino acid sequence as set forth in any one of SEQ ID NOs.: 14, 24, 26, 28, 38, 40, 42, 58, 60, 66, and 68, for example, any one amino acid sequence that has at least 95%, at least 96%, at least 97%, at least 98%, or at least 99% homology to the amino acid sequence as set forth in any one of SEQ ID NOs.: 14, 24, 26, 28, 38, 40, 42, 58, 60, 66, and 68.

In some cases, the second polypeptide chain may comprise an amino acid sequence as set forth in any one of SEQ ID NOs.: 14, 24, 28, 38, 40, 42, 58, 60, 66, and 68. In some cases, the second polypeptide chain may comprise an amino acid sequence that has at least 90% homology to an amino acid sequence as set forth in any one of SEQ ID NOs.: 14, 24, 28, 38, 40, 42, 58, 60 and 68, for example, any one amino acid sequence that has at least 95%, at least 96%, at least 97%, at least 98%, or at least 99% homology to the amino acid sequence as set forth in any one of SEQ ID NOs.: 14, 24, 28, 38, 40, 42, 58, 60 and 68.

In the present application, the fusion protein may comprise a first polypeptide chain and a second polypeptide chain, wherein the first IgG Fc domain of the first polypeptide chain may interact with the second IgG Fc domain of the second polypeptide chain to form a dimer.

In the present application, in the fusion protein, the first complement inhibitory domain of the first polypeptide chain may be identical to the second complement inhibitory domain of the second polypeptide chain. For example, the first complement inhibitory domain and the second complement inhibitory domain may comprise a protein selected from the group consisting of factor H (FH), CD46, CD55, CD59 and CR1, or a functional fragment thereof. For example, the first complement inhibitory domain and the second complement inhibitory domain may comprise a protein selected from the group consisting of factor H (FH), CD55 and CD59, or a functional fragment thereof.

In some cases, in the fusion protein, the first IgG Fc domain of the first polypeptide chain may be identical to the second IgG Fc domain of the second polypeptide chain. For example, the first IgG Fc domain and/or the second IgG Fc domain may comprise an amino acid sequence as set forth in any one of SEQ ID NOs.: 10 and 30. For example, the first IgG Fc domain and/or the second IgG Fc domain may comprise an amino acid sequence that has at least 90% homology to an amino acid sequence as set forth in any one of SEQ ID Nos.: 10 and 30, for example, any one amino acid sequence that has at least 95%, at least 96%, at least 97%, at least 98%, or at least 99% homology to the amino acid sequence as set forth in any one of SEQ ID NOs.: 10 and 30.

For example, the fusion protein may comprise a first polypeptide chain and a second polypeptide chain, and the first polypeptide chain may be identical to the second polypeptide chain. For example, the first polypeptide chain and/or the second polypeptide chain may comprise an amino acid sequence as set forth in any one of SEQ ID NOs.: 14, 24, 26, 28, 58 and 60. For example, the first polypeptide chain and/or the second polypeptide chain may comprise an amino acid sequence as set forth in any one of SEQ ID NOs.: 14, 24 and 28. For example, the first polypeptide chain and/or the second polypeptide chain may comprise an amino acid sequence that has at least 90% homology to an amino acid sequence as set forth in any one of SEQ ID NOs.: 14, 24, 26, 28, 58 and 60, for example, any one amino acid sequence that has at least 95%, at least 96%, at least 97%, at least 98%, or at least 99% homology to the amino acid sequence as set forth in any one of SEQ ID NOs.: 14, 24, 26, 28, 58 and 60.

In the present application, in the fusion protein, the first complement inhibitory domain of the first polypeptide chain may be different from the second complement inhibitory domain of the second polypeptide chain. For example, the first complement inhibitory domain and the second complement inhibitory domain each independently comprise a protein selected from the group consisting of CD59 and CD55, or a functional fragment thereof. For example, the first complement inhibitory domain and the second complement inhibitory domain each independently comprise a protein selected from the group consisting of FH and CD55, or a functional fragment thereof. For example, the first complement inhibitory domain and the second complement inhibitory domain each independently comprise a protein selected from the group consisting of CD46 and CD59, or a functional fragment thereof.

In some cases, in the fusion protein, the first IgG Fc domain of the first polypeptide chain may be identical to the second IgG Fc domain of the second polypeptide chain. For example, the first IgG Fc domain and/or the second IgG Fc domain may comprise an amino acid sequence as set forth in any one of SEQ ID NOs.: 10 and 30.

In some cases, in the fusion protein, the first IgG Fc domain of the first polypeptide chain may be different from the second IgG Fc domain of the second polypeptide chain. For example, the first IgG Fc domain may comprise an amino acid sequence as set forth in SEQ ID NO.: 32, and the second IgG Fc domain may comprise an amino acid sequence as set forth in SEQ ID NO.: 34.

For example, the first polypeptide chain or the second polypeptide chain of the fusion protein may comprise the CRIg extracellular domain, CD46, and IgG1 Fc in sequence from the N-terminus to the C-terminus. For example, the first polypeptide chain may comprise an amino acid sequence as set forth in SEQ ID NO: 36.

For example, the first polypeptide chain or the second polypeptide chain of the fusion protein may comprise the CRIg extracellular domain, CD55, and IgG1 Fc in sequence from the N-terminus to the C-terminus. For example, the first polypeptide chain may comprise an amino acid sequence as set forth in SEQ ID NO: 38.

For example, the second polypeptide chain or the second polypeptide chain of the fusion protein may comprise the CRIg extracellular domain, FH, and IgG1 Fc in sequence from the N-terminus to the C-terminus. The second polypeptide chain may comprise an amino acid sequence as set forth in SEQ ID NO: 40.

For example, the second polypeptide chain or the second polypeptide chain of the fusion protein may comprise the CRIg extracellular domain, CD59, and IgG1 Fc in sequence from the N-terminus to the C-terminus. The second polypeptide chain may comprise an amino acid sequence as set forth in SEQ ID NO: 42.

For example, the first polypeptide chain may comprise an amino acid sequence as set forth in any one of SEQ ID NOs.: 36, 38, 40 and 42.

For example, the first polypeptide chain may comprise an amino acid sequence as set forth in any one of SEQ ID NOs.: 38, 40 and 42.

For example, the second polypeptide chain may comprise an amino acid sequence as set forth in any one of SEQ ID NOs.: 36, 38, 40 and 42.

For example, the second polypeptide chain may comprise an amino acid sequence as set forth in any one of SEQ ID NOs.: 38, 40 and 42.

For example, the first polypeptide chain of the fusion protein may comprise an amino acid sequence as set forth in SEQ ID NO: 38, and the second polypeptide chain may comprise an amino acid sequence as set forth in any one of SEQ ID NOs: 40 and 42.

For example, the first polypeptide chain of the fusion protein may comprise an amino acid sequence as set forth in SEQ ID NO: 38; and the second polypeptide chain may comprise an amino acid sequence as set forth in SEQ ID NO: 40.

For example, the first polypeptide chain of the fusion protein may comprise an amino acid sequence as set forth in SEQ ID NO: 38; and the second polypeptide chain may comprise an amino acid sequence as set forth in SEQ ID NO: 42.

For example, the first polypeptide chain of the fusion protein may comprise an amino acid sequence as set forth in SEQ ID NO: 36; and the second polypeptide chain may comprise an amino acid sequence as set forth in SEQ ID NO: 42.

In the present application, the first polypeptide chain, the second polypeptide chain, the CRIg extracellular domain, the complement inhibitory domain (for example, the factor H (FH), CD46, CD55 and CD59), and the synergetic domain (for example, the IgG Fc domain and/or human serum albumin and/or the fusion protein) comprise not only the respective amino acid sequences described above, but may also comprise variants of the respective amino acid sequences.
In the present application, the variants of amino acid sequences may comprise: 1) an amino acid sequence having at least 90% (for example, at least 91%, at least 92%, at least 93%, at least 94%, at least 95%, at least 96%, at least 97%, at least 98%, at least 99%, or at least 100%) sequence homology to the corresponding amino acid sequence; and/or
2) an amino acid sequence obtained by substitution, deletion, or addition of one or more (for example, 1 to 2, 1 to 3, 1 to 4, 1 to 5, 1 to 6, 1 to 7, 1 to 8, 1 to 9, 1 to 10, 1 to 11, 1 to 12 or more) amino acids to the corresponding amino acid sequence.

In the present application, the term "homology" generally refers to sequence similarity or interchangeability between two or more polynucleotide sequences or between two or more polypeptide sequences. When a computer program or software (for example, Emboss Needle or BestFit) is used to determine the sequence identity, similarity, or homology between different amino acid sequences, default parameter settings can be used. An appropriate score matrix, such as blosum45 or blosum80, can also be selected to optimize identity, similarity, or homology scores. In some embodiments, homologous polynucleotides comprise polynucleotides that are capable of hybridizing to a control polynucleotide sequence under stringent conditions and have at least 60%, at least 65%, at least 70%, at least 80%, at least 90%, at least 95%, at least 97%, at least 98%, at least 99%, or even at least 100% sequence identity compared to the control polynucleotide sequence. The homologous polypeptides may be polypeptides that have at least 80%, at least 85%, at least 90%, at least 95%, at least 97%, at least 98%, at least 99%, or even at least 100% sequence identity to a control polypeptide sequence when sequence alignment is performed under optimized conditions.

To determine sequence identity, sequence alignment may be performed by various means known to those skilled in the art, for example, by using BLAST, BLAST-2, ALIGN, NEEDLE, or Megalign (DNASTAR) software, etc. Those skilled in the art are able to determine appropriate parameters for the alignment, including any algorithm required to achieve optimal alignment among the full-length sequences being aligned.

In the present application, the amino acid substitution may be a conserved amino acid substitution or a non-conserved amino acid substitution. After substitution, the first polypeptide chain, the second polypeptide chain, the CRIg extracellular domain, the complement inhibitory domain (for example, the factor H (FH), CD55 and CD59) and/or the synergetic domain (for example, the IgG Fc domain and/or human serum albumin) still have the same or similar functional activity as/to the first polypeptide chain, the second polypeptide chain, the CRIg extracellular domain, the complement inhibitory domain (for example, the factor H (FH), CD55 and CD59) and/or the synergetic domain (for example, the IgG Fc domain and/or human serum albumin) before the substitution.

For example, the amino acid substitution may be non-conserved substitution. The non-conserved substitution may comprise changing an amino acid residue in a protein or polypeptide of interest in a non-conserved form, for example, changing an amino acid residue having certain side chain size or certain property (for example, hydrophilicity) to an amino acid residue having a different side chain size or a different property (for example, hydrophobicity).

The amino acid substitution may also be conserved substitution. The conserved substitution may comprise changing an amino acid residue in a protein or polypeptide of interest in a conserved form, for example, changing an amino acid residue having certain side chain size or certain property (for example, hydrophilicity) to an amino acid residue having the same or similar side chain size or the same or similar property (for example, still hydrophilicity). Such a conserved substitution typically has no significant impact on the structure or function of the resulting protein. In the present application, the variants of the amino acid sequence(s) of the first polypeptide chain, the second polypeptide chain, the CRIg extracellular domain, the complement inhibitory domain (for example, the factor H (FH), CD46, CD55, CD59, and CR1) and/or the synergetic domain (for example, the IgG Fc domain and/or human serum albumin) may involve conserved amino acid substitutions that do not significantly change the protein structure or its function.

By way of example, the mutual substitutions between individual amino acids within each of the following groups may be considered as conserved substitutions in the present application: a group of amino acids having non-polar side chains: alanine, valine, leucine, isoleucine, proline, phenylalanine, tryptophan, and methionine;
a group of uncharged amino acids with polar side chains: glycine, serine, threonine, cysteine, tyrosine, asparagine, and glutamine;
a group of negatively charged amino acids with polar side chains: aspartic acid and glutamic acid;
positively charged basic amino acids: lysine, arginine, and histidine; and
amino acids with a phenyl group: phenylalanine, tryptophan, and tyrosine.

### Nucleic Acid Molecule, Vector and Cell

In another aspect, the present application further provides an isolated nucleic acid molecule or isolated nucleic acid molecules. The nucleic acid molecule or nucleic acid molecules may encode the fusion protein of the present application or a fragment thereof. For example, the nucleic acid molecule or nucleic acid molecules may encode the entire fusion protein (for example, when the fusion protein is single-chain), or encode a portion of the fusion protein, for example,the first polypeptide chain, the second polypeptide chain, the CRIg extracellular domain, the complement inhibitory domain (for example, the factor H (FH), CD46, CD55, CD59, and CR1) and/or the synergetic domain (for example, the IgG Fc domain and/or human serum albumin), or one or more thereof.

In the present application, the nucleic acid molecule may comprise a nucleotide sequence as set forth in any one of SEQ ID NOs.: 13, 15, 23, 25, 27, 35, 37, 39, 41, 57, 59, 65, and 67.

The nucleic acid molecule of the present application may be isolated. For example, it can be produced or synthesized by the following methods: (i) *in vitro* amplification, for example by polymerase chain reaction (PCR) amplification, (ii) clonal recombination, (iii) purification, for example, by fractionation through restriction digestion and gel electrophoresis, or (iv) synthesis, for example, by chemical synthesis. In some embodiments, the isolated nucleic acid(s) is/are a nucleic acid molecule(s) prepared by the recombinant DNA technology.

In the present application, the nucleic acid encoding the fusion protein or the fragment thereof can be prepared by a variety of methods known in the art. These methods include, but are not limited to, the overlap extension PCR using restriction fragment operations or using synthetic oligonucleotides. For specific operations, see Sambrook et al., Molecular Cloning, A Laboratory Manual, Cold Spring Harbor Laboratory Press, Cold Spring Harbor, N.Y., 1989; and Ausube et al., Current Protocols in Molecular Biology, Greene Publishing and Wiley-Interscience, New York N.Y., 1993.

In another aspect, the present application provides a vector or vectors, each of which comprises the nucleic acid molecule or nucleic acid molecules of the present application. Each vector may comprise the nucleic acid molecule or nucleic acid molecules. In addition, the vector may also comprise other genes, for example, a marker gene that is allowed to select this vector in a suitable host cell and under a suitable condition. In addition, the vector may also comprise an expression control element that allows a coding region to be expressed correctly in a suitable host. Such a control element is well known to those skilled in the art, which, for example, may include a promoter, a ribosome binding site, an enhancer, and other control elements that regulate gene transcription or mRNA translation. In some embodiments, the expression control sequence is a regulatable element. A specific structure of the expression control sequence may vary depending on the function of the species or cell type, but generally includes a 5' non-transcribed sequence and 5' and 3' non-translated sequences, for example, a TATA box, a capped sequence, a CAAT sequence, etc., which are involved in transcription and translation initiation, respectively. For example, the 5' non-transcribed expression control sequence may include a promoter region, and the promoter region may include a promoter sequence for functionally linked to the nucleic acid for transcriptional control. The expression control sequence may further comprise an enhancer sequence or an upstream activator sequence. In the present application, suitable promoters may comprise, for example, promoters for SP6, T3, and T7 polymerases, human U6 RNA promoters, CMV promoters, and their artificial hybrid promoters (such as CMV), wherein a portion of a promoter may be fused with a portion of a promoter of an additional cellular protein (such as human GAPDH and glyceraldehyde-3-phosphate dehydrogenase) gene, and the promoter may or may not contain additional introns. The nucleic acid molecule or nucleic acid molecules of the present application may be operably linked to the expression control element. The vector may comprise, for example, a plasmid, a cosmid, a virus, a bacteriophage, or other vectors commonly used in, for example, genetic engineering. For example, the vector is an expression vector.

In another aspect, the present application provides a host cell, which may comprise the nucleic acid molecule or nucleic acid molecules of the present application and/or the vector or vectors of the present application. In some embodiments, each type of or each host cell may comprise one or one type of the nucleic acid molecule or vector of the present application. In some embodiments, each type of or each cell may comprise a plurality of (for example, 2 or more) or a plurality of types of (for example, 2 or more types of) vectors of the present application. For example, the vector of the present application may be introduced into the host cell, for example, an eukaryotic cell, such as a plant-originated cell, a fungal cell, or a yeast cell, etc. The vector of the present application may be introduced into the host cell by methods known in the art, such as electroporation, lipofectine transfection, lipofectamin transfection, etc.

### Pharmaceutical Composition

In another aspect, the present application provides a pharmaceutical composition comprising the fusion protein and an optionally pharmaceutically acceptable carrier. The pharmaceutically acceptable carrier generally refers to a carrier for preparing a pharmaceutical composition or formulation. It is typically safe and non-toxic, and is neither biologically nor otherwise undesirable. The carrier used is typically one that is suitable for administration to a human body or other mammals. In the preparation of a composition, an active ingredient is usually mixed with the carrier and diluted or enclosed by the carrier. When the carrier is used as a diluent, it may be a solid, semisolid or liquid material that acts as a mediator, carrier or medium for the active ingredient of the antibody. The pharmaceutically acceptable carrier may include a buffer, an antioxidant, a preservative, a low molecular weight polypeptide, a protein, a hydrophilic polymer, an amino acid, a sugar, a chelating agent, a counterion, a metal complex and/or a non-ionic surfactant, etc.

In the present application, the pharmaceutical composition may be formulated for oral administration, intravenous administration, intramuscular administration, in situ administration at a tumor site, inhalation, rectal administration, vaginal administration, transdermal administration, or administration via a subcutaneous depot. A solution or suspension for transdermal administration or administered via a subcutaneous depot may comprise the following components: sterile diluents, such as water for injection, a saline solution, nonvolatile oil, polyethylene glycol, glycerol, propylene glycol, or other synthetic solvents; antimicrobial agents, such as benzyl alcohol or methyl parahydroxybenzoate; antioxidants, such as ascorbic acid or sodium bisulfite; chelating agents, such as ethylenediaminetetraacetic acid (EDTA); buffers, such as acetate, citrate, or phosphate; and tension-regulating substances, such as sodium chloride or dextrose. pH can be adjusted with an acid or a base, for example, hydrochloric acid or sodium hydroxide.

### Methods and Uses

In another aspect, the present application provides a preparation method of the fusion protein or the fragment thereof. The method may comprise the steps of: synthesizing the fusion protein or the fragment thereof, and/or, culturing the cell under a condition of allowing the expression of the fusion protein or the fragment thereof. For example, the medium, temperature, culture time and the like may be used as appropriate, and these methods are understood by those of ordinary skills in the art.

The present application may utilize the genetic engineering technology to link the fusion protein of the present application and the fragment thereof, for example, the first polypeptide chain, the second polypeptide chain, the CRIg extracellular domain, the complement inhibitory domain (for example, the factor H (FH), CD46, CD55, CD59, and CR1) and/or the synergetic domain (for example, the IgG Fc domain and/or human serum albumin), or to link the amino acid residues in sequence based on the sequences of proteins.

The present application may also utilize the genetic engineering technology to link the encoding sequences of the fusion protein of the present application and the fragment thereof, for example, the first polypeptide chain, the second polypeptide chain, the CRIg extracellular domain, the complement inhibitory domain (for example, the factor H (FH), CD46, CD55, CD59, and CR1) and/or the synergetic domain (for example, the IgG Fc domain and/or human serum albumin), orto link the bases in sequence based on the sequences of nucleic acids.

In another aspect, the present application provides use of the fusion protein or the pharmaceutical composition in the preparation of a drug for treatment of diseases associated with targeted inhibition of complement activation. The diseases associated with targeted inhibition of complement activation may be selected from: paroxysmal nocturnal hemoglobinuria (PNH), atypical aaemolytic uraemic syndrome (aHUS), generalized myasthenia gravis (gMG), neuromyelitis optica spectrum disorders (NMOSD), age-related macular degeneration (AMD), autoimmune hemolytic anemia, autoimmune thrombocytopenia, aplastic anemia, systemic lupus erythematosus, rheumatoid arthritis, ankylosing spondylitis, atherosclerosis, Parkinson's disease, Alzheimer's disease (senile dementia), asthma, allergies, psoriasis, multiple sclerosis, and Crohn's disease. The drug of the present application can inhibit the inhibition of complement activation and/or protect cells from complement attacks. In some cases, the diseases may comprise autoimmune diseases. For example, the diseases may comprise autoimmune myasthenia gravis.

Not wishing to be bound by any particular theory, the following examples are merely to illustrate the fusion protein, preparation methods and applications and the like according to the present application, and are not intended to limit the scope of the present invention. ns: no significance, *p<0.05, **p<0.01, ***p<0.001, and ****p<0.0001.

### Examples

### Example 1: Extension of drug half-life of complement inhibitor by addition of synergetic domain fragment

Experimental objective: By introducing human IgG Fc or HSA into a CRIg-FH complement inhibitor, constructing expression vectors for two recombinant proteins, (CRIg-FH-IgG4Fc)×2 and His×6-CRIg-FH-HSA, and performing eukaryotic expression and purification to detect their complement inhibitory activity and *in vivo* half-life, it was intended to ultimately screen and determine protein fragments capable of extending the half-life of CRIg-FH without affecting its complement inhibitory effect.

### 1. Instruments and materials:

Electric thermostatic water tank (Model DK-8D, Shanghai JingHong Laboratory Instrument Co., Ltd.), PCR instrument (Mastercycler pro-Eppendorf, Eppendorf, Germany), RNA/DNA concentration/purity tester (NANODROP 2000c, Thermo Scientific, USA), gel imager (Tanon 1600, Shanghai Tanon Technology Co., Ltd.), CO₂ cell incubator (240i, Thermo Scientific, USA), BioRAD Mini protein Tera system (BioRAD, USA), and low-temperature horizontal centrifuge (Allegra X-15R Centrifuge, Beckman Coulter, USA).

### 2. Experimental methods:

### 2.1 Gene cloning and vector construction

Total RNAs were extracted from human hepatoma cell lines Hep3B using NucleoZOL (Macherey-Nagel, Germany), and reversely transcribed into cDNAs using reverse transcriptases (PrimeScript^{™} RT Master Mix, Takara, Japan). These cDNAs were amplified, by PCR using corresponding primers, for the gene sequences encoding the SCR1-5 domains (E19-K323) of the FH complement inhibition functional fragment and the human serum albumin (HSA). In addition, with a similar method, total RNAs were extracted from lymphoma cells U937, reversely transcribed into cDNAs, and then amplified by PCR for the gene sequences encoding the CRIg gene extracellular domains (G19-K137).

The nucleic acid sequence encoding the FH protein was as set forth in SEQ ID NO.: 3, and the amino acid of the FH protein was as set forth in SEQ ID NO.: 4; and the nucleic acid sequence encoding the SCR1-5 domains of FH was as set forth in SEQ ID NO.: 7, and the amino acid sequence of the SCR1-5 domains of FH was as set forth in SEQ ID NO.: 8.

The nucleic acid sequence encoding the HSA protein was as set forth in SEQ ID NO.: 11, and the amino acid sequence of the HSA protein was as set forth in SEQ ID NO.: 12.

The nucleic acid sequence encoding the CRIg protein was as set forth in SEQ ID NO.: 1, and the amino acid sequence of the CRIg protein was as set forth in SEQ ID NO.: 2. The nucleic acid sequence encoding the CRIg extracellular domain was as set forth in SEQ ID NO.: 5, and the amino acid sequence of the CRIg extracellular domain was as set forth in SEQ ID NO.: 6.

Primers containing the sequences of both CRIg extracellular domains and FH SCR1-5 domains were designed, in which CRIg extracellular domain genes were linked to FH SCR1-5 genes by the overlapping PCR, and inserted in pFUSE-hIgG4-Fc1 eukaryotic expression vectors (InvivoGen). The nucleic acid sequence encoding hIgG4-Fc was as set forth in SEQ ID NO.: 9, and the amino acid sequence of the hIgG4-Fc protein was as set forth in SEQ ID NO.: 10. In addition, the above CRIg extracellular domain genes and the FH SCR1-5 after linkage were linked to the HSA genes by the overlapping PCR, and then inserted into pcDNA3.1/His A (Invitrogen) expression vectors. The above vectors were sequenced in both directions to confirm that the correct insertion sequences were identified for subsequent experiments, and the vectors were named pFUSE-CRIg-FH-hIgG4Fc 1 and pCDNA/His-CRIg-FH-HSA, respectively.

### 2.2 Protein expression

At a density of 2.0×10⁷ cells/dish, 293FT cells were evenly spread in a cell culture dish the diameter of 15 cm; after growing to the logarithmic phase, the cells were transfected with the above constructed large plasmids of PFUSE-CRIg-FH-hIgG4-Fc1 or pCDNA/His-CRIg-FH-HSA by using the reagent Lipofectamine 2000 (Invitrogen, USA); the cells were cultured for 6 hours in a 5% CO₂ incubator at 37°C, and then, the medium was replaced with a 293 protein expression serum-free medium (Gibco, USA); and after three days of consecutive culture, the cell culture supernatant was collected and then centrifuged to remove the cells and cell debris, and the resulting supernatant was concentrated by using an ultrafiltration tube before purification.

### 2.3 Protein affinity purification, buffer replacement and concentration

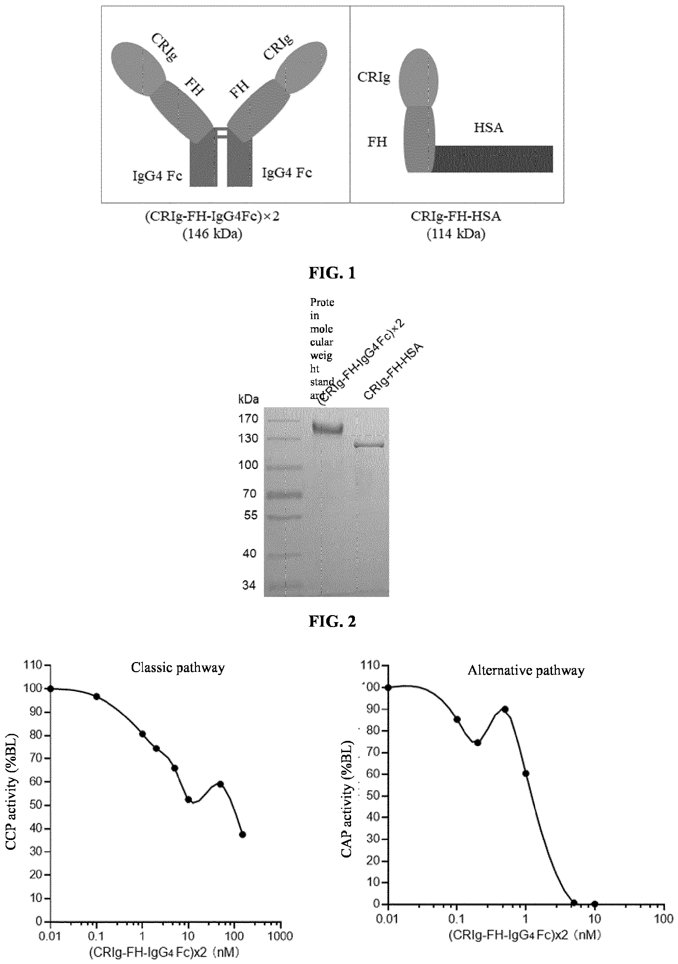

With the Protein A antibody purification magnetic bead kit (Beaver Biosciences Inc., Suzhou, China), the (CRIg-FH-IgG4Fc)×2 recombinant proteins in the cell supernatant were purified and eluted into the elution buffer, and then transferred to an ultrafiltration tube (Millipore, USA); and the buffer was replaced with PBS by low-temperature centrifugation, and the proteins were concentrated. Or, with the His•Bind affinity Purification Kit (Novagen/MerckMillipore), the expressed His×6-CRIg-FH-HSA fusion proteins were purified according to the manufacturing instructions, and the buffer was replaced with a PBS by a similar method and concentrated for storage. The resulting recombinant proteins were named as (CRIg-FH-IgG4Fc)×2 and CRIg-FH-HSA, respectively (FIG. 1).

### 2.4 Complement inhibitory activity assay

The above two recombinant proteins were tested in terms of their inhibitory effects on the classical and alternative pathways of complements by using the commercially available kits WIESLAB^{®} Complement System Classical Pathway (COMPLCP310) and WIESLAB^{®} Complement Alternative Pathway (COMPLAP330), respectively.

### 2.5 In vivo drug half-life assay of recombinant proteins

SD rats, with an even mix of male and female, were injected intravenously with (CRIg-FH-IgG4Fc)×2; the blood was collected before administration (D-1), and 5 min (i.e., 2 min after the end of administration), 30 min, 4h, 24h, 2d, 3d, 5d, 7d, 10d, 14d, 21d and 28d after the start of administration D1, respectively, and serum was separated. The concentration of recombinant proteins in each sample was analyzed by ELISA. Or, CRIg-FH-HSA was injected intravenously at 1 mg/kg, and the serum was separated before drug administration, and 5 min, 30 min, 1h, 3h, 6h, 12h, 24h, 2d, 3d, 5d, 7d, 10d, 14d, 21d, and 28d after the start of administration, respectively. Or, CRIg-FH (with the preparation method from the published paper of the inventors, Qiao Q., et al., A novel CRIg-targeted complement inhibitor protects cells from complement damage, FASEB J. 2014 Nov;28(11):4986-99.) was injected intravenously at 20 mg/kg, and the serum was separated before drug administration, and 5 min, 30 min, 1h, 3h, 6h, 12h, 18h, 24h, 36h, 48h, 72h, and 120h after administration, respectively.

ELISA was performed as follows: the encapsulated rabbit anti-human CRIg monoclonal antibody (Clone No.: 202, sino biological, Catalog# 12163-H08H) bound to recombinant proteins in standard or in serum, and then (CRIg-FH-IgG4Fc)×2 was detected by mouse anti-human IgG4 fragment Secondary (5c7) [HRP] (NOVUS., Catalog# NB110-7081H), or CRIg-FH-HSA was detected by human serum albumin polyclonal antibody, HRP (ThermoFisher, Catalog# PA1-72058), or CRIg-FH was detected by HRP-labeled rabbit anti-human FH monoclonal antibody EPR6225 (Catalog# ab133536). The blood concentrations of recombinant proteins in serum were calculated by standard curves, time-drug serum concentration curves were plotted, and half-lives were calculated.

### 3. Experimental results:

3.1 Based on the above method, two recombinant proteins, (CRIg-FH-IgG4Fc)×2 and His×6-CRIg-FH-HSA, were obtained by applying the eukaryotic system for induced expression and purification, and were detected by PAGE electrophoresis, with the purity greater than 95%. Their theoretical molecular weights were 146 kDa and 114 kDa, respectively, and the actual values matched with the theoretical values (FIG. 2). The nucleic acid sequence encoding CRIg-FH-IgG4 Fc was as set forth in SEQ ID NO.: 13, and the amino acid sequence of the CRIg-FH-IgG4 Fc protein was as set forth in SEQ ID NO.: 14; the nucleic acid sequence encoding CRIg-FH-HSA was as set forth in SEQ ID NO.: 15, and the amino acid sequence of the CRIg-FH-HSA protein was as set forth in SEQ ID NO.: 16; and the nucleic acid sequence encoding CRIg-FH was as set forth in SEQ ID NO.: 51, and the amino acid sequence of the CRIg-FH protein was as set forth in SEQ ID NO.: 52.

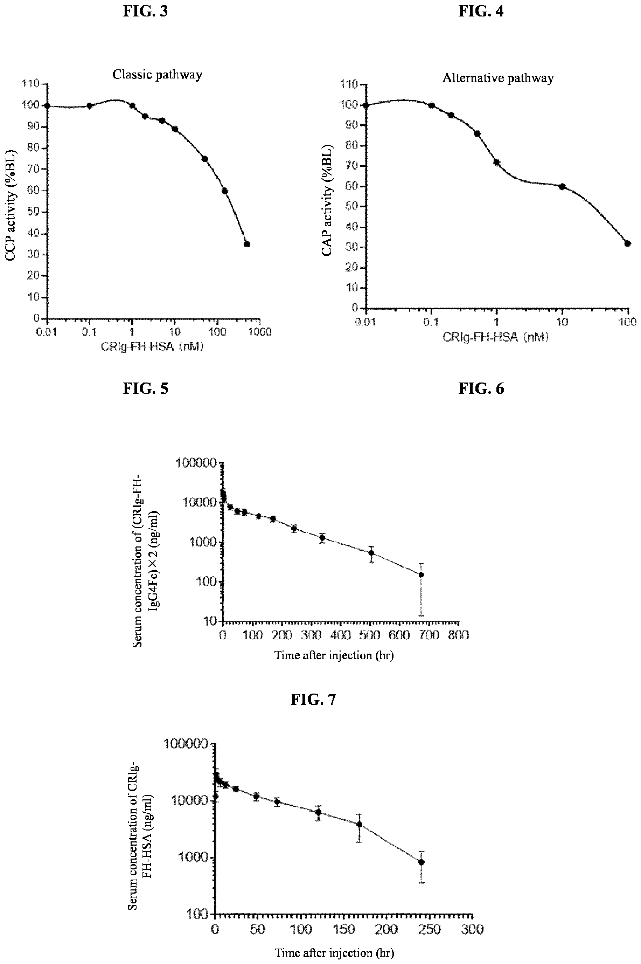

3.2 We tested the inhibitory activities of (CRIg-FH-IgG4 Fc)×2 and CRIg-FH-HSA against the classical and alternative pathways of human serum complements. The results showed that both (CRIg-FH-IgG4 Fc)×2 and CRIg-FH-HSA had inhibitory effects on complements. The former inhibited the classical pathway of complement at an IC50 of 91.38 nM (FIG. 3), and the alternative pathway of complement at an IC50 of 1.04 nM (FIG. 4); and the latter inhibited the classical pathway of complement at an IC50 of 1355 nM (FIG. 5), and the alternative pathway of complement at an IC50 of 10.39 nM (FIG. 6).

### 3.3 (CRIg-FH-IgG4 Fc)×2 and CRIg-FH-HSA

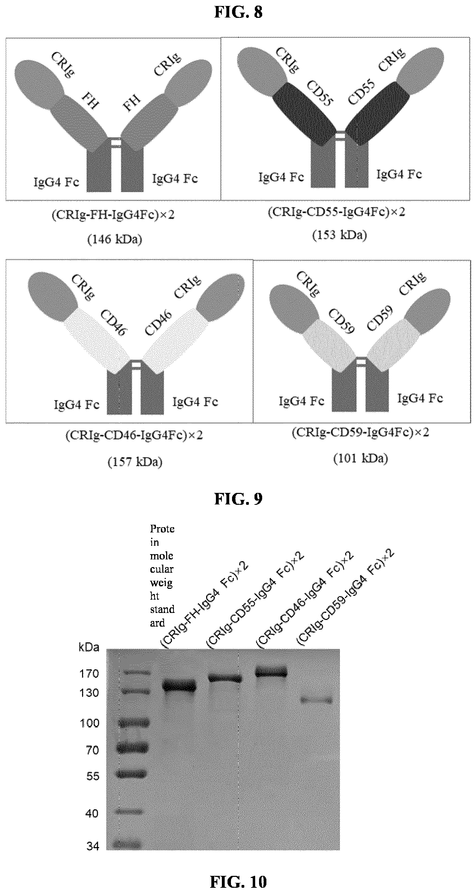

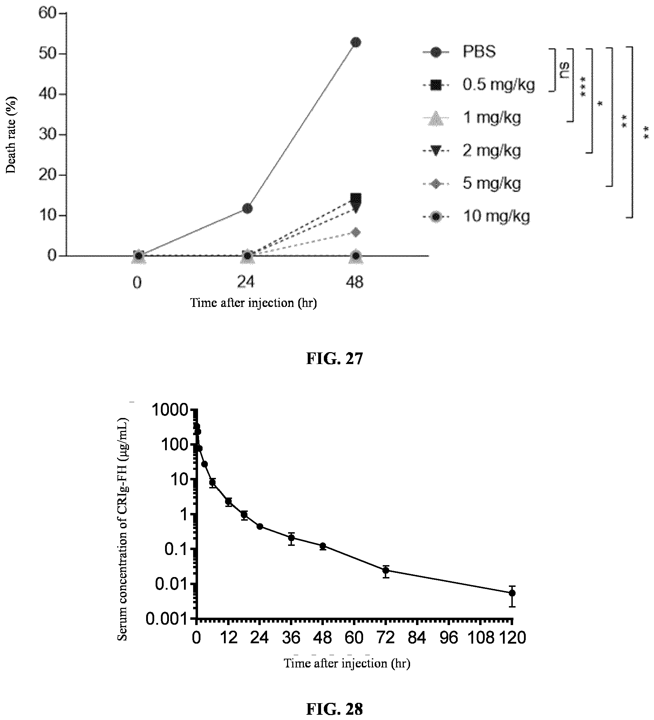

The concentration of (CRIg-FH-IgG4 Fc)×2 or CRIg-FH-HSA or CRIg-FH in serum collected at different time points were measured, and the time-concentration pharmacokinetic curves were plotted, in which the pharmacokinetic curve of (CRIg-FH-IgG4 Fc)×2 was shown in FIG. 7, with a half-life of 142.2 hours; the pharmacokinetic curve of CRIg-FH-HSA was shown in FIG. 8, with a half-life of 32.8 hours; and the pharmacokinetic curve of CRIg-FH was shown in FIG. 28, with a half-life of 0.56 hours. These results indicate that the addition of the synergetic domain (for example, the Fc fragment of the antibody or HSA) significantly extends the half-life of CRIg-FH by more than 250 times and more than 58 times, respectively.

### Example 2 Excellent complement inhibitory effect resulting from linkage of complement inhibitory domain to CRIg

Experimental objective: By constructing the expression vectors of a total of nine recombinant proteins, including (CRIg-FH-IgG4Fc)×2, (CRIg-CD55-IgG4Fc)×2, (CRIg-CD46-IgG4Fc)×2, (CRIg-CD59-IgG4Fc)×2, (CRIg-CR1-IgG4Fc)×2, (CRIg-IgG4Fc)×2, (FH-IgG4Fc)×2, (FH-CRIg-IgG4Fc)×2, and (CRIg-L-FH-IgG4Fc)×2, and performing eukaryotic expression and purification, and detecting their complement activities, it was intended to determine which inhibitory proteins showed an excellent complement inhibitory effect after these proteins were linked to CRIg in five important complement inhibitory proteins FH, CD55, CD46, CD59 and CR1, and to determine the impact of the linkage form between the complement inhibitory domain and the synergetic domain on the complement inhibitory activity.

### 1. Instruments and materials:

Electric thermostatic water tank (Model DK-8D, Shanghai JingHong Laboratory Instrument Co., Ltd.), PCR instrument (Mastercycler pro-Eppendorf, Eppendorf, Germany), RNA/DNA concentration/purity tester (NANODROP 2000c, Thermo Scientific, USA), gel imager (Tanon 1600, Shanghai Tanon Technology Co., Ltd.), CO₂ cell incubator (240i, Thermo Scientific, USA), BioRAD Mini protein Tera system (BioRAD, USA), and low-temperature horizontal centrifuge (Allegra X-15R Centrifuge, Beckman Coulter, USA).

### 2. Experimental methods:

### 2.1 Gene cloning and vector construction

Total RNAs were extracted from human normal pancreatic ductal epithelial cells HPDE6-C7 using NucleoZOL (Macherey-Nagel, Germany), and reversely transcribed into cDNAs using reverse transcriptases (PrimeScript^{™} RT Master Mix, Takara, Japan). These cDNAs were amplified, by PCR using corresponding primers, for the DNA sequences (all excluding DNA sequences encoding signal peptides) of CD55, CD46, and CD59. With a similar method, total RNAs were extracted from cells of human hepatoma cell lines Hep3B by using NucleoZOL, reversely transcribed into cDNAs, and then amplified by PCR for the gene sequences encoding SCR1-5 domains (E19-K323) of the FH complement inhibition functional fragments; and total RNAs were extracted from lymphoma cells U937, reversely transcribed into cDNAs, and then amplified by PCR for the gene sequences encoding CRIg gene extracellular domains (G19-K137) as well as CR1 CCP8-11 and CCP15-18 domains. The nucleic acid sequence encoding CD55 was as set forth in SEQ ID NO.: 17, and the amino acid sequence of CD55 was as set forth in SEQ ID NO.: 18; the nucleic acid sequence encoding CD46 was as set forth in SEQ ID NO.: 19, and the amino acid sequence of the CD46 protein was as set forth in SEQ ID NO.: 20; the nucleic acid sequence encoding CD59 was as set forth in SEQ ID NO.: 21, and the amino acid sequence of the CD59 protein was as set forth in SEQ ID NO.: 22; and the nucleic acid sequences encoding CR1 CCP8-11 and CCP15-18 were as set forth in SEQ ID NOs.: 61 and 63, respectively, and the protein sequences of CR1 CCP8-11 and CCP15-18 were as set forth in SEQ ID NOs.: 62 and 64, respectively.

Primers containing the encoding sequences of the CRIg extracellular domain and a flexible linker peptide (Gly4Ser)₃, the CRIg extracellular domain and/or the FH SCR1-5 domain (specifically, there were four forms expressed as: the CRIg extracellular domain alone, the FH SCR1-5 alone, the CRIg extracellular domain at the N-terminus and the FH SCR1-5 at the C-terminus, and the FH SCR1-5 at the N-terminus and the CRIg extracellular domain at the C-terminus), CD55, CD46, CD59 or CR1 (CCP15-18 domains) at the same time were designed. The single CRIg extracellular domain, the single FH SCR1-5, the CRIg extracellular domain gene and FH SCR1-5 with or without the flexible linker peptide attached, CD46, CD55, CD59 or CR1 CCP15-18 genes were linked by the overlapping PCR, and inserted into the pFUSE-hIgG4-Fc1 eukaryotic expression vector (InvivoGen). The above vectors were sequenced in both directions to confirm that the correct insertion sequences were identified for subsequent experiments, and the vectors were named pFUSE-CRIg-L-FH-IgG4Fc, pFUSE-CRIg-IgG4Fc, pFUSE-FH-IgG4Fc, pFUSE-FH-CRIg-IgG4Fc, pFUSE-CRIg-FH-IgG4Fc, pFUSE-CRIg-CD55-IgG4Fc, pFUSE-CRIg-CD46-IgG4Fc, pFUSE-CRIg-CD59-IgG4Fc, and pFUSE-CRIg-CR1-IgG4Fc, respectively.

### 2.2 Protein expression

At a density of 2.0×10⁷ cells/dish, 293FT cells were evenly spread in a cell culture dish with the diameter of 15 cm; after growing to the logarithmic phase, the cells were transfected with the above constructed large plasmids of pFUSE-CRIg-L-FH-IgG4Fc, pFUSE-CRIg-IgG4Fc, pFUSE-FH-IgG4Fc, pFUSE-FH-CRIg-IgG4Fc, pFUSE-CRIg-FH-IgG4Fc, pFUSE-CRIg-CD55-IgG4Fc, pFUSE-CRIg-CD46-IgG4Fc, pFUSE-CRIg-CD59-IgG4Fc, or pFUSE-CRIg-CR1-IgG4Fc by using the reagent Lipofectamine 2000 (Invitrogen, USA); the cells were cultured for 6 hours in a 5% CO₂ incubator at 37°C, and then, the medium was replaced with a 293 protein expression serum-free medium (Gibco, USA); and after three days of consecutive culture, the cell culture supernatant was collected and then centrifuged to remove the cells and cell debris, and the resulting supernatant was concentrated by using an ultrafiltration tube before purification.

### 2.3 Protein affinity purification, buffer replacement and concentration

With the Protein A antibody purification magnetic bead kit (Beaver Biosciences Inc., Suzhou, China), the recombinant proteins (CRIg-L-FH-IgG4Fc)×2, (CRIg-IgG4Fc)×2, (FH-IgG4Fc)×2, (FH-CRIg-IgG4Fc)×2, (CRIg-FH-IgG4Fc)×2, (CRIg-CD55-IgG4Fc)×2, (CRIg-CD46-IgG4Fc)×2, (CRIg-CD59-IgG4Fc)×2, or (CRIg-CR1-IgG4Fc)×2 in the cell supernatant were purified and eluted into the elution buffer, and then transferred to an ultrafiltration tube (Millipore, USA); and the buffer was replaced with PBS by low-temperature centrifugation, and the proteins were concentrated for storage.

### 2.4 Complement inhibitory activity assay

The above recombinant proteins were tested in terms of their inhibitory effects on the classical and alternative pathways of complements by using the commercially available kits WIESLAB^{®} Complement System Classical Pathway (COMPLCP310) and WIESLAB^{®} Complement Alternative Pathway (COMPLAP330), respectively.

### 3. Experimental results:

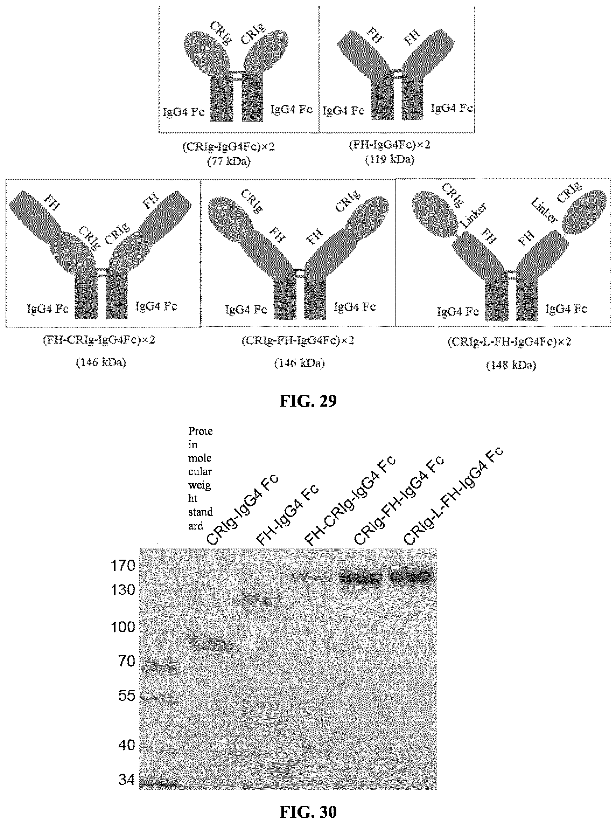

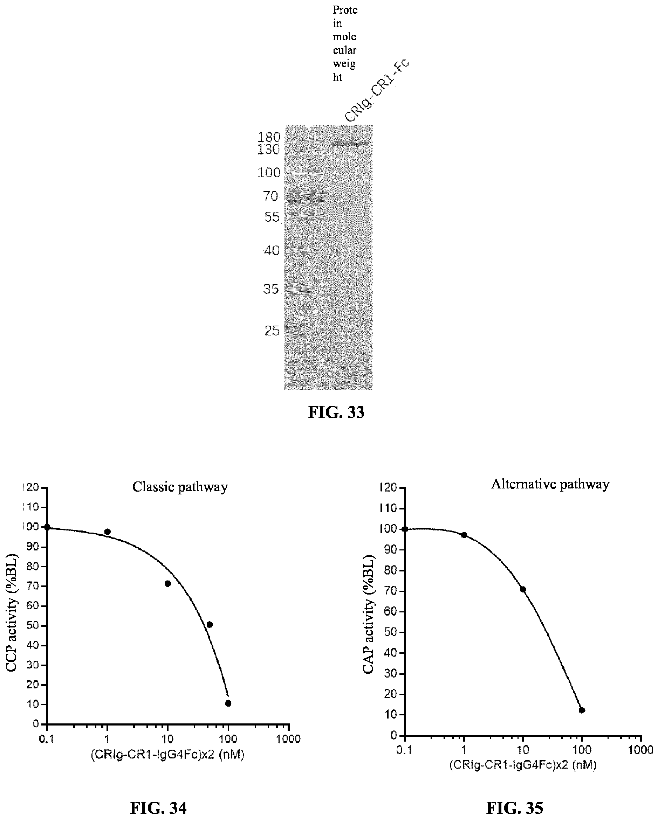

3.1 Based on the above method, nine recombinant proteins, (CRIg-IgG4Fc)×2, (FH-IgG4Fc)×2, (FH-CRIg-IgG4Fc)×2, (CRIg-L-FH-IgG4Fc)×2, (CRIg-FH-IgG4Fc)×2, (CRIg-CD55-IgG4Fc)×2, (CRIg-CD46-IgG4Fc)×2, (CRIg-CD59-IgG4Fc)×2, and (CRIg-CR1-IgG4Fc)×2 (see FIG. 9, FIG. 29, and FIG. 32 for their structures), were obtained by applying the eukaryotic system for induced expression and purification, and were detected by PAGE electrophoresis, with the purity greater than 95%. Their theoretical molecular weights were 77 kDa, 119 kDa, 146 kDa, 146 kDa and 148 kDa (FIG. 30), 146 kDa, 153 kDa, 157 kDa and 101 kDa (FIG. 10), and 134 kDa (FIG. 33), respectively, and the actual values matched with the theoretical values.

The nucleic acid sequence encoding CRIg-IgG4 Fc was as set forth in SEQ ID NO.: 53, and the amino acid sequence of CRIg-IgG4 Fc was as set forth in SEQ ID NO.: 54; the nucleic acid sequence encoding FH-IgG4 Fc was as set forth in SEQ ID NO.: 55, and the amino acid sequence of FH-IgG4 Fc was as set forth in SEQ ID NO.: 56; the nucleic acid sequence encoding FH-CRIg-IgG4 Fc was as set forth in SEQ ID NO.: 57, and the amino acid sequence of FH-CRIg-IgG4 Fc was as set forth in SEQ ID NO.: 58; the nucleic acid sequence encoding CRIg-L-FH- IgG4 Fc was as set forth in SEQ ID NO.: 59, and the amino acid sequence of CRIg-L-FH-IgG4 Fc was as set forth in SEQ ID NO.: 60; the nucleic acid sequence encoding CRIg-CD55-IgG4Fc was as set forth in SEQ ID NO.: 23, and the amino acid sequence of a CRIg-CD55-IgG4Fc protein was as set forth in SEQ ID NO.: 24; the nucleic acid sequence encoding CRIg-CD46-IgG4Fc was as set forth in SEQ ID NO.: 25, and the amino acid sequence of a CRIg-CD46-IgG4Fc protein was as set forth in SEQ ID NO.: 26; the nucleic acid sequence encoding CRIg-CD59-IgG4Fc was as set forth in SEQ ID NO.: 27, and the amino acid sequence of a CRIg-CD59-IgG4Fc protein was as set forth in SEQ ID NO.: 28; and the nucleic acid sequence encoding CRIg-CR1(CCP15-18)-IgG4Fc was as set forth in SEQ ID NO.: 67, and the amino acid sequence of the CRIg-CR1(CCP15-18)-IgG4Fc protein was as set forth in SEQ ID NO.: 68.

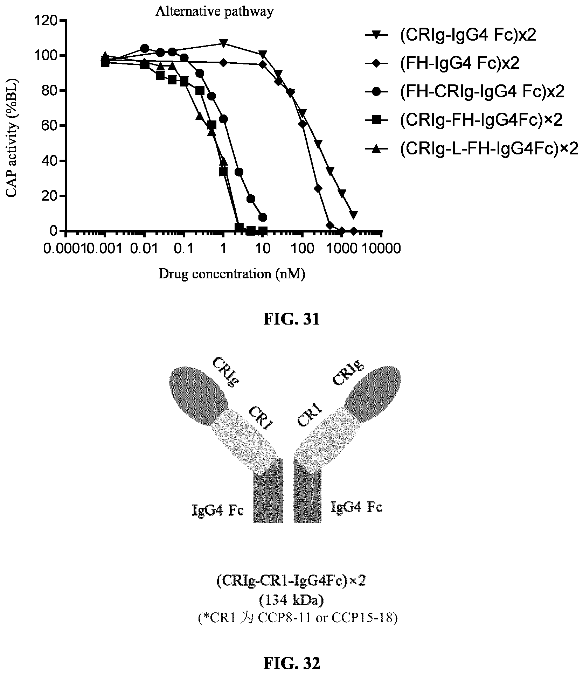

3.2 We tested the inhibitory activity of five recombinant proteins, (CRIg-IgG4 Fc)×2, (FH-IgG4 Fc)×2, (FH-CRIg-IgG4Fc)×2, (CRIg-FH-IgG4Fc)×2 and (CRIg-L-FH-IgG4Fc)×2, against the alternative pathway of human serum complements, and the inhibitory activity of five recombinant proteins, (CRIg-FH-IgG4Fc)×2, (CRIg-CD55-IgG4Fc)×2, (CRIg-CD46-IgG4Fc)×2, (CRIg-CD59-IgG4Fc)×2 and (CRIg-CR1-IgG4Fc)×2, against the classical and alternative pathways of human serum complements. The inhibitory effects of five recombinant proteins, (CRIg-IgG4 Fc)×2, (FH-IgG4 Fc)×2, (FH-CRIg-IgG4Fc)×2, (CRIg-FH-IgG4Fc)×2, and (CRIg-L-FH-IgG4Fc)×2, against the alternative pathway of human serum complements can be found in FIG. 31, specifically with the IC50s of 248.4 nM, 138.8 nM, 1.48 nM, 0.75 nM, and 0.69 nM (Table 1), respectively. These results indicate that, firstly, the complement inhibitory effect of CRIg nor FH alone is not as good as that of the linkage of two, and the CRIg linked to either the N-terminus or C-terminus of the FH can enhance the complement inhibitory effect; secondly, when the two are linked, CRIg located at the N-terminus of FH shows a complement inhibitory effect slightly better than that when it is located at the C-terminus of FH; and finally, the complement inhibitory effect can be enhanced by linking the CRIg to the FH either directly or via a linker such as the flexible linker peptide (Gly4Ser)₃.

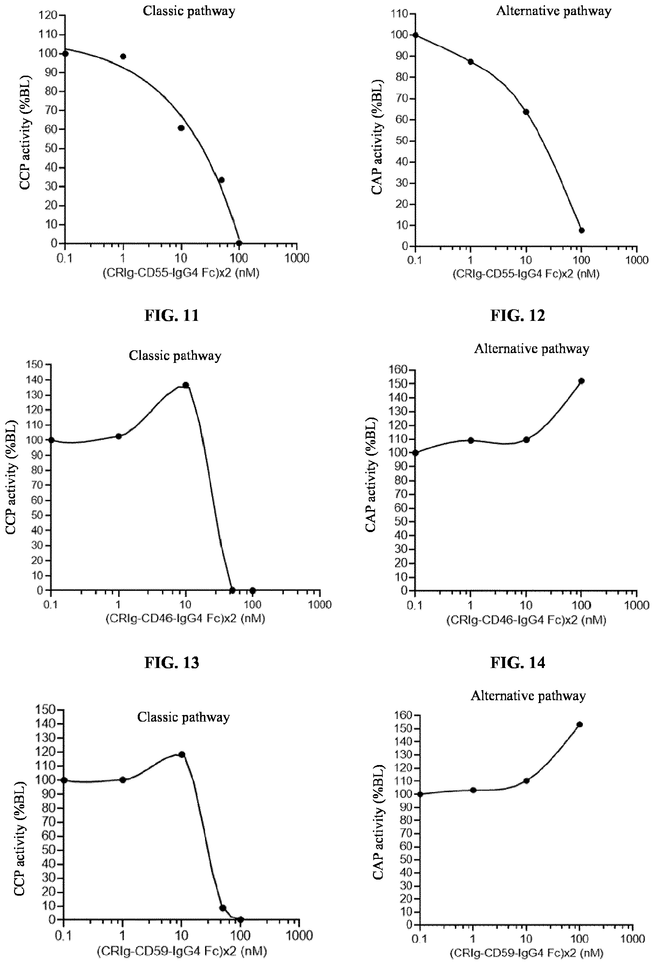

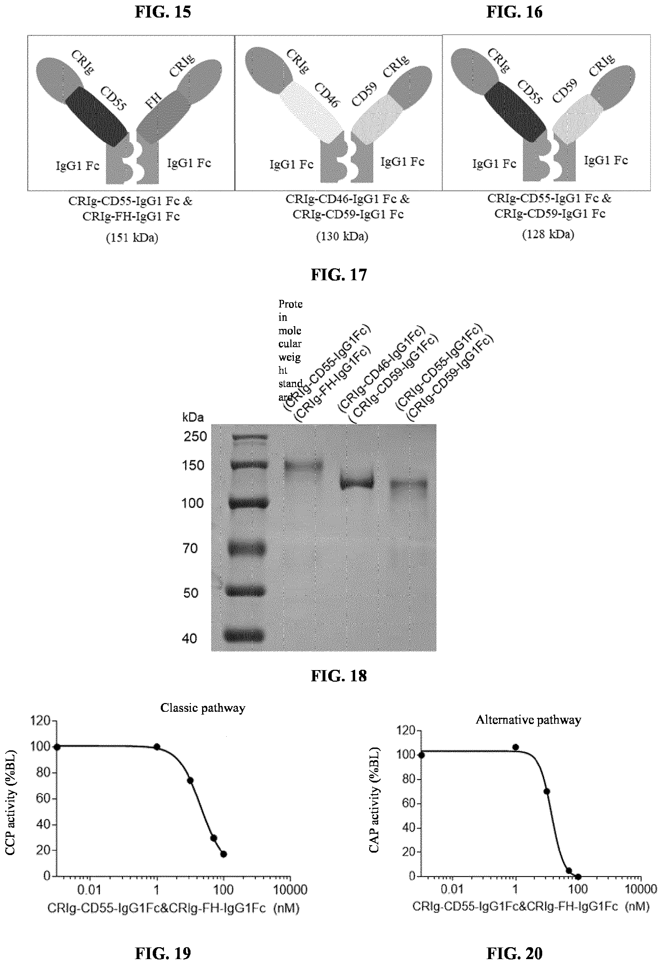

In addition, the inhibitory effects of (CRIg-FH-IgG4 Fc)×2 against the classical and alternative pathways of complements can be found in FIG. 3 and FIG. 4, respectively; the inhibitory effects of (CRIg-CD55-IgG4Fc)×2 against the classical and alternative pathways of complements can be found in FIG. 11 and FIG. 12, respectively; the inhibitory effects of (CRIg-CD46-IgG4Fc)×2 against the classical and alternative pathways of complements can be found in FIG. 13 and FIG. 14, respectively; the inhibitory effects of (CRIg-CD59-IgG4Fc)×2 against the classical and alternative pathways of complements can be found in FIG. 15 and FIG. 16, respectively; and the inhibitory effects of (CRIg-CR1-IgG4Fc)×2 against the classical and alternatve pathways of complements can be found in FIG. 34 and FIG. 35, respectively. Their IC50s for the inhibition of the classical pathway of complements are detailed in Table 2. Specifically, the IC50s of (CRIg-FH-IgG4 Fc)×2 for the inhibition of the classical and alternative pathways of complements are 91.38 nM and 1.04 nM, respectively (where the difference between the two test results of the inhibitory activity against the alternative pathway, i.e., 0.75 nM *vs.* 1.04 nM, may be related to different test batches); the IC50s of (CRIg-CD55-IgG4Fc)×2 for the inhibition of the classical and alternative pathways of complements are 23.25 nM and 19.66 nM, respectively; the IC50 of (CRIg-CD46-IgG4Fc)×2 for the inhibition of the classical pathway of complements is 44.06 nM; the IC50 of (CRIg-CD59-IgG4Fc)×2 for the inhibition of the classical pathway of complements is 44.84 nM; and the IC50s of (CRIg-CR1-IgG4Fc)×2 for the inhibition of the classical and alternative pathways of complements are 35.75 nM and 30.26 nM, respectively. All five fusion proteins show the inhibitory effect against the classical and/or alternative pathways of complements.

**Table 1 Comparison of in vitro complement inhibitory effects of complement inhibitors**

| Name of complement inhibitor | IC50 for inhibition of alternative pathway (nM) |
|---|---|
| (CRIg-IgG4 Fc)×2 | 248.4 |
| (FH-IgG4 Fc)×2 | 138.8 |
| (FH-CRIg-IgG4 Fc)×2 | 1.48 |
| (CRIg-FH-IgG4Fc)×2 | 0.75 |
| (CRIg-L-FH-IgG4Fc)×2 | 0.69 |

### Example 3 Development of bispecific targeted complement inhibitors

Experimental objective: By constructing the expression vectors of a total of three bispecific targeted recombinant proteins (CRIg-CD55-IgG1 Fc)(CRIg-FH-IgG1Fc), (CRIg-CD46-IgG1 Fc)(CRIg-CD59-IgG1 Fc), and (CRIg-CD55-IgG1 Fc)(CRIg-CD59-IgG1 Fc), performing eukaryotic expression and purification on these fusion proteins, and detecting their complement inhibitory activities, it was intended to screen more effective complement inhibitors.

### 1. Instruments and materials:

Electric thermostatic water tank (Model DK-8D, Shanghai JingHong Laboratory Instrument Co., Ltd.), PCR instrument (Mastercycler pro-Eppendorf, Eppendorf, Germany), RNA/DNA concentration/purity tester (NANODROP 2000c, Thermo Scientific, USA), gel imager (Tanon 1600, Shanghai Tanon Technology Co., Ltd.), CO₂ cell incubator (240i, Thermo Scientific, USA), BioRAD Mini protein Tera system (BioRAD, USA), and low-temperature horizontal centrifuge (Allegra X-15R Centrifuge, Beckman Coulter, USA).

### 2. Experimental methods:

### 2.1 Gene cloning and vector construction

By designing mutagenic primers, the mutation sites of primers for pFUSE-hIgG1-Fc1 eukaryotic expression vectors were modified into (Alegre et al., 1992; Carter, 2001; Merchant et al., 1998; Ridgway et al., 1996; Xu et al., 2000) two mutant vectors, pFUSE-hIgG1-Fc1 knob mutant and pFUSE-hIgG1- Fc1 hole mutant, by using a point mutation kit (TOYOBO, Japan).

The nucleic acid sequence encoding IgG1 Fc was as set forth in SEQ ID NO.: 29, and the amino acid sequence of the IgG1 Fc protein was as set forth in SEQ ID NO.: 30; the nucleic acid sequence encoding the Knob mutant was as set forth in SEQ ID NO.: 31, and the amino acid sequence of the Knob mutant protein was as set forth in SEQ ID NO.: 32; and the nucleic acid sequence encoding the Hole mutant was as set forth in SEQ ID NO.: 33; and the amino acid sequence of the Hole mutant protein was as set forth in SEQ ID NO.: 34.

Based on the CRIg-FH, CRIg-CD46, CRIg-CD55 and CRIg-CD59 gene sequences constructed in Examples 1 and 2, the fusion fragments CRIg-CD46, CRIg-CD55 were inserted into the pFUSE-hIgG1-Fc knob mutant vector using a one-step rapid cloning kit (Shanghai Yeasen Biotechnology Co., Ltd.), with the plasmids named pFUSE-CRIg-CD46-hIgG1 Fc knob, and pFUSE-CRIg-CD55-hIgG1 Fc knob; and the fusion fragments CRIg-FH and CRIg-CD59 were inserted into the pFUSE-IgG1-Fc hole mutant vector by using the one-step rapid cloning kit, with the plasmids named pFUSE-CRIg-FH-IgG1 Fc hole and pFUSE-CRIg-CD59-IgG1 Fc hole, respectively.

The nucleic acid sequence encoding the CRIg-CD46-IgG1 Fc knob was as set forth in SEQ ID NO.:35, and the amino acid sequence of the CRIg-CD46-IgG1 Fc knob protein was as set forth in SEQ ID NO.: 36; the nucleic acid sequence encoding the CRIg-CD55-IgG1 Fc knob was as set forth in SEQ ID NO.: 37, and the amino acid sequence of the CRIg-CD55-IgG1 Fc knob protein was as set forth in SEQ ID NO.: 38; the nucleic acid sequence encoding the CRIg-FH-IgG1 Fc hole was as set forth in SEQ ID NO.: 39, and the amino acid sequence of the CRIg-FH-IgG1 Fc hole protein was as set forth in SEQ ID NO.: 40; and the nucleic acid sequence encoding the CRIg-CD59-IgG1 Fc hole was as set forth in SEQ ID NO.: 41, and the amino acid sequence of the CRIg-CD59-IgG1 Fc hole protein was as set forth in SEQ ID NO.: 42.

### 2.2 Protein expression

At a density of 2.0×10⁷ cells/dish, 293FT cells were evenly spread in a cell culture dish with the diameter of 15 cm; after growing to the logarithmic phase, the cells were transfected with large plasmids of pFUSE-CRIg-CD46-hIgG1 Fc knob and pFUSE-CRIg-CD59-hIgG1 Fc hole, as well as pFUSE-CRIg-CD55-hIgG1 Fc knob and pFUSE-CRIg-CD59-hIgG1 Fc hole, pFUSE-CRIg-CD55-hIgG 1 Fc knob and pFUSE-CRIg-FH-hIgG1 Fc hole by using the reagent Lipofectamine 2000 (Invitrogen, USA); the cells were cultured for 6 hours in a 5% CO₂ incubator at 37°C, and then, the medium was replaced with a 293 protein expression serum-free medium (Gibco, USA); and after three days of consecutive culture, the cell culture supernatant was collected and then centrifuged to remove the cells and cell debris, and the resulting supernatant was concentrated by using an ultrafiltration tube before purification.

### 2.3 Protein affinity purification, buffer replacement and protein concentration

With the Protein A antibody purification magnetic bead kit (Beaver Biosciences Inc., Suzhou, China), the recombinant proteins (CRIg-CD46-IgG1 Fc) (CRIg-CD59-IgG1 Fc), (CRIg-CD55-IgG1 Fc) (CRIg-CD59-IgG1 Fc), and (CRIg-CD55-IgG1 Fc) (CRIg-FH-IgG1 Fc) in the cell supernatant were purified and eluted into the elution buffer, and then transferred to an ultrafiltration tube (Millipore, USA); and the buffer was replaced with a PBS by low-temperature centrifugation, and the proteins were concentrated.

### 2.4 Complement inhibitory activity assay

The above two recombinant proteins were tested in terms of their inhibitory effects on the classical and alternative pathways of complements by using the commercially available kits WIESLAB^{®} Complement System Classical Pathway (COMPLCP310) and WIESLAB^{®} Complement Alternative Pathway (COMPLAP330), respectively. The data was analyzed using Graphpad Prism 7.0 software.

### 3. Experimental results:

3.1 Based on the above method, three recombinant proteins, (CRIg-CD55-IgG1 Fc) (CRIg-FH-IgG1 Fc), (CRIg-CD46-IgG1 Fc) (CRIg-CD59-IgG1 Fc), and (CRIg-CD55-IgG1 Fc) (CRIg-CD59-IgG1 Fc) (FIG. 17), were obtained by applying the eukaryotic system for induced expression and purification, and were detected by PAGE electrophoresis, with the purity greater than 95%. Their theoretical molecular weights were 151 kDa, 130 kDa, and 128 kDa, respectively, and the actual values matched with the theoretical values (FIG. 18).

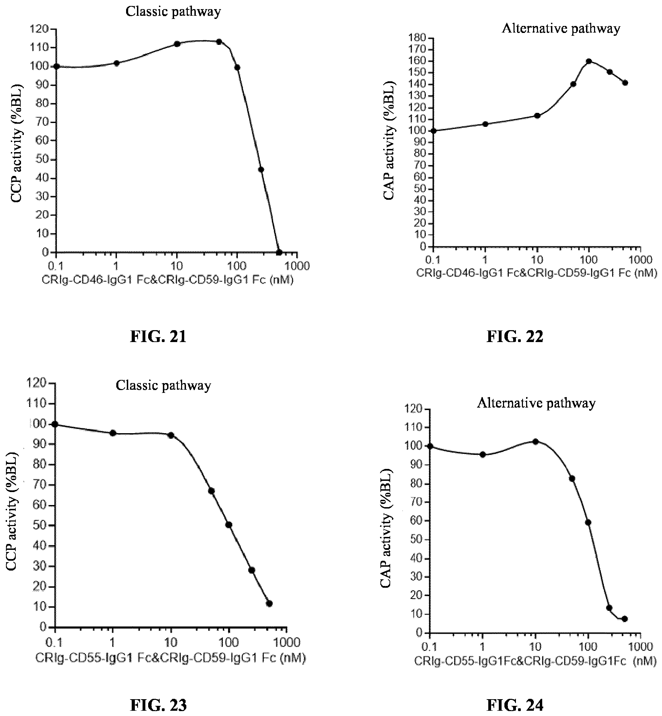

3.2 The IC50s of inhibitory activities of the three bispecific targeted complement inhibitors against the classical and alternative pathways of complements are shown in Table 2. The inhibitory effects of (CRIg-CD55-IgG1 Fc) (CRIg-FH-IgG1 Fc) against the classical and alternative pathways of complements are as shown in FIG. 19 and FIG. 20, respectively; the inhibitory effects of (CRIg-CD46-IgG1 Fc) (CRIg-CD59-IgG1 Fc) against the classical and alternative pathways of complements are as shown in FIG. 21 and FIG. 22, respectively; and the inhibitory effects of (CRIg-CD55-IgG1 Fc) (CRIg-CD59-IgG1 Fc) against the classical and alternative pathways of complements are as shown in FIG. 23 and FIG. 24, respectively. All three bispecific targeted complement inhibitors are capable of inhibiting the classical or alternative pathway of complements.

**Table 2 Comparison of in vitro complement inhibitory effects of complement inhibitors**

| Name of complement inhibitor | IC50 for inhibition of classical pathway (nM) | IC50 for inhibition of alternative pathway (nM) |
|---|---|---|
| (CRIg-FH-IgG4 Fc)×2 | 91.38 | 1.04 |
| CRIg-FH-HSA | 1335 | 10.39 |
| (CRIg-CD55-IgG4 Fc)×2 | 23.25 | 19.66 |
| (CRIg-CD46-IgG4 Fc)×2 | 44.06 | greater than 500 |
| (CRIg-CD59-IgG4 Fc)×2 | 44.84 | greater than 500 |
| (CRIg-CR1-IgG4 Fc)×2 | 35.75 | 30.26 |
| (CRIg-CD55-IgG1 Fc) | 20.8 | 14.1 |
| (CRIg-FH-IgG1 Fc) | | |
| (CRIg-CD46-IgG1 Fc) | 238.7 | greater than 500 |
| (CRIg-CD59-IgG1 Fc) | | |
| (CRIg-CD55-IgG1 Fc) | 115.4 | 111.6 |
| (CRIg-CD59-IgG1 Fc) | | |

### Example 4 Efficacy of (CRIg-FH-IgG4Fc)×2 in the treatment of rat myasthenia gravis model

Experimental objective: Among the aforementioned fusion proteins, we selected the (CRIg-FH-IgG4Fc)×2 complement inhibitor to test the efficacy in the treatment of diseases associated with excessive complement activation *in vivo.* The expression level of AChR was directly downregulated by binding the autoantibody against the acetylcholine receptor (AChR) to AChR at the neuromuscular junction, or the dysfunction or decreased expression level of AChR was indirectly induced by activating the classical pathway of complement, both leading to neuromuscular conduction disorders and eventually clinical manifestations of MG such as myasthenia (Gomez et al., 2010; Phillips and Vincent, 2016). We tested the therapeutic effect of the (CRIg-FH-IgG4Fc)×2 complement inhibitor in a rat myasthenia gravis (experimental autoimmune myasthenia gravis (EAMG)) model, to lay the foundation for potential subsequent clinical applications.

### 1. Instruments and materials:

Animal weighing balance (Shanghai Precision Scientific Instruments Co., Ltd., YP2001N), Lewis rats (Beijing Vital River Laboratory Animal Technology Co., Ltd.), and InVivo MAb anti-human/rat/fish AChR (Bio X Cell, West Lebanon, NH).

### 2. Experimental methods:

### 2.1 Induction of EAMG

Intraperitoneal administration of anti-AChR antibody mAb35 (1-3 mg/kg) to female rats aged about four weeks is a universal international method for the induction of a generalized myasthenia gravis-like experimental autoimmune myasthenia gravis (EAMG) model (Hepburn et al., 2007; Liu et al., 2007; Papanastasiou et al., 2000; Poulas et al., 2000). Rats were divided into a normal group without injection of the anti-AChR antibody, a group with intraperitoneal injection of the anti-AChR antibody (1.5 mg/kg) and PBS, and treatment groups with intraperitoneal injection of the anti-AChR antibody and the (CRIg-FH-IgG4Fc)×2 drug at 5 different doses (0.5, 1, 2, 5 and 10 mg/kg), 7 groups in total, for which two experiment were completed. 40 rats were for the first experiment and 50 rats for the second experiment. The groups are shown in Table 3.

**Table 3 Grouped observation of efficacy of (CRIg-FH-IgG4Fc)×2 drug against rat EAMG induced by anti-AChR antibodies**

| Gro up no. | Group name | Number of animals | | Intraperiton eal injection of anti-AChR antibodies | Intraperitoneal injection of treatment protocol |
|---|---|---|---|---|---|
| | | First experi ment | Second experime nt | | |
| 1 | Normal group | 5 | - | PBS | PBS |
| 2 | Untreated model group | 7 | 10 | 1.5 mg/kg | PBS |
| 3 | Model treatment group 1 | 7 | - | 1.5 mg/kg | drug, 0.5 mg/kg |
| 4 | Model treatment group 2 | 7 | 10 | 1.5 mg/kg | drug, 1 mg/kg |
| 5 | Model treatment group 3 | 7 | 10 | 1.5 mg/kg | drug, 2 mg/kg |
| 6 | Model treatment group 4 | 7 | 10 | 1.5 mg/kg | drug, 5 mg/kg |
| 7 | Model treatment group 5 | - | 10 | 1.5 mg/kg | drug, 10 mg/kg |

### 2.2 Monitoring of the EAMG phenotype

Rats were experimented after an adaptation period of at least 5-7 days. They were weighed every 24 hours before and after each experiment, and clinically scored. The specific scoring criteria are as follows: 0 point in case of capability of grasping and lifting the cage cover; 1 point in case of capability of grasping but incapability of lifting the cage cover; 2 points in case of incapability of grasping the cage cover; 3 points in case of incapability of grasping an object and hind limb paralysis; and 4 points in case of death or a near death state (Piddlesden et al., 1996; Soltys et al., 2009). In the experiments, it was found that most of the rats in the PBS control group among the model groups died or faced death at 48 hours, and thus, the observation for this group was ended at the 48-hour time point according to the ethics. The treatment group with the (CRIg-FH-IgG4Fc)×2 drug had basically recovered their clinical scores at 96 hours, and thus, the observation for this group was ended at the 96-hour time point, and the animal death rates at 24 and 48 hours were counted.

### 2.3 Statistical treatment

Experimental data were represented as mean ± standard deviation (Mean ± SEM), the body weight was analyzed by the two tails t-test, the clinical scores were analyzed by the two-way ANOVA, and the death rate was determined by the Chi-Square and Fisher's exact test, with ns: no significance, *p<0.05, **p<0.01, ***p<0.001, and ****p<0.0001.

### 3. Experimental results:

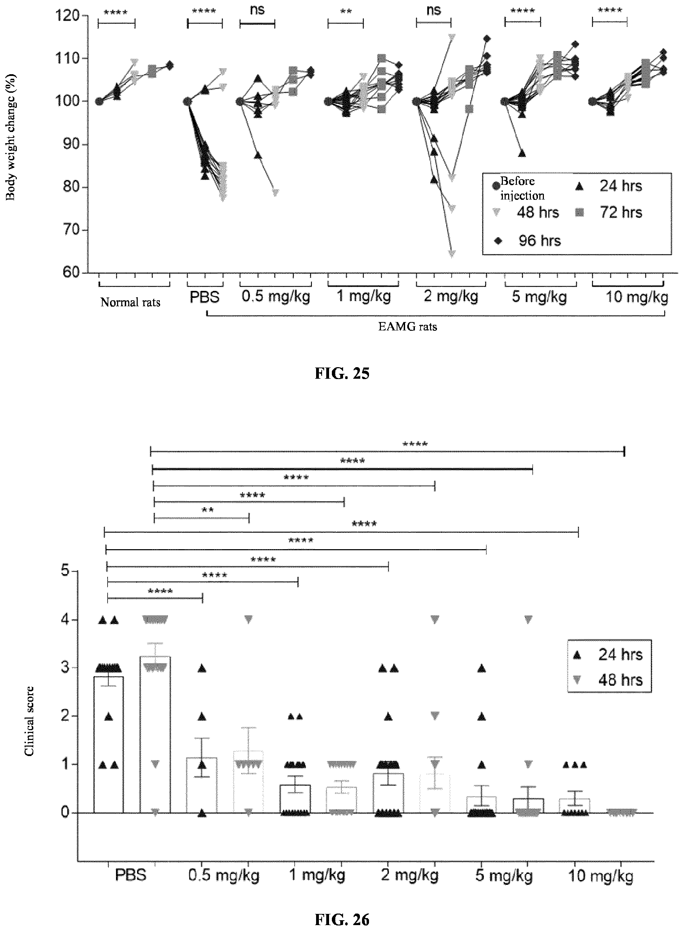

The normal rats without injection of the anti-AChR antibody and (CRIg-FH-IgG4Fc)×2 drug gradually gained weight. The rats with injection of the anti-AChR antibody and without drug treatment lost 11.2% of their body weight at 24 hours and 15.5% at 48 hours, with the clinical scores of 3-4 at 24 hours accounting for 82% (14/17) and 3-4 at 48 hours accounting for 88% (15/17). Moreover, among the 17 rats, 2 rats were found to be dying or dead at 24 hours and 9 rats were found to be dead at 48 hours, with a death rate of 52.9% (9/17), and 6 of the remaining 8 surviving animals also approached the near death state, indicating that the EAMG rat model was successfully constructed (FIG. 25 to FIG. 27).

After pretreatment with the (CRIg-FH-IgG4Fc)×2 drug, the symptoms of EAMG were significantly ameliorated. After treatment at the lowest drug dose of 0.5 mg/kg, the body weight decreased by only 1.6% at 24 hours, with a clinical score of 1.1, and by only 2.3% at 48 hours, with a clinical score of 1.3, and the animal death rate was 14.3% (1/7), indicating a significant efficacy. After treatment with the drug at a higher dose, the body weight was still not lost at 24 hours, and the clinical score gradually decreased in a dose-dependent way; and at 48 hours, the body weight gradually recovered, and the clinical score also gradually decreased, showing certain dose-efficacy relationship. That is, the higher the dose, the more the body weight increased, and the lower the clinical score (FIG. 25 to FIG. 27). In addition, all the animals in the drug treatment groups were observed up to 96 hours, and at 48 hours, the surviving rats showed a gradual increase in body weight and a gradual decrease of the clinical scores to normal values, indicating that the EAMG symptoms gradually disappear.

### 4. Discussions:

The EAMG phenotype can be induced by intraperitoneal injection of the anti-AChR antibody mAb35 (1.5 mg/kg) to female rats aged about four weeks, with the manifestations of significant weight loss, rapid loss of locomotivity, loss of forelimb grip, and animal death within 48 hours. Accordingly, the model is successfully constructed. The results of body weight monitoring, clinical scoring and animal death monitoring on the rats show that LM007 exerts a significant EAMG treatment effect at a drug dose of 0.5 mg/kg, showing a dose-dependent relationship between efficacy and dose as the dose increases, with almost complete blockage of the disease progression at 5 mg/kg and above. In the rat EAMG model induced by the anti-AChR antibody, excessive complement activation predominates, and very few rats may undergo a pathogenic action without depending on complement activation.

## Claims

1. A fusion protein, comprising:
(i) a CRIg extracellular domain;
(ii) a complement inhibitory domain, comprising a protein selected from the group consisting of factor H (FH), CD55, CD46, CD59 and CR1, or a functional fragment thereof; and
(iii) a synergetic domain, comprising a protein selected from the group consisting of an IgG Fc domain and a human serum albumin, or a functional fragment thereof.

2. The fusion protein according to claim 1, wherein said CRIg extracellular domain comprises an amino acid sequence as set forth in SEQ ID NO.: 6.

3. The fusion protein according to any one of claims 1 to 2, wherein a C-terminus of said CRIg extracellular domain is directly or indirectly linked to an N-terminus of said complement inhibitory domain, or, an N-terminus of said CRIg extracellular domain is directly or indirectly linked to a C-terminus of said complement inhibitory domain.

4. The fusion protein according to any one of claims 1 to 3, wherein the C-terminus of said complement inhibitory domain is directly or indirectly linked to an N-terminus of said synergetic domain, or, wherein the C-terminus of said CRIg extracellular domain is directly or indirectly linked to the N-terminus of said synergetic domain.

5. The fusion protein according to any one of claims 1 to 4, wherein said indirect linking is performed via a linker.

6. The fusion protein according to claim 5, wherein said linker comprises an amino acid sequence as set forth in any one of SEQ ID NOs.: 44, 46, 48, and 50.

7. The fusion protein according to any one of claims 1 to 6, wherein said complement inhibitory domain comprises an amino acid sequence as set forth in any one of SEQ ID NOs.: 8, 18, 20, 22, 62, and 64.

8. The fusion protein according to any one of claims 1 to 7, wherein said synergetic domain is a human serum albumin.

9. The fusion protein according to any one of claims 1 to 8, wherein said human serum albumin comprises an amino acid sequence as set forth in SEQ ID NO.: 12.

10. The fusion protein according to any one of claims 1-9, which has a single-chain structure.

11. The fusion protein according to any one of claims 1-10, which comprises said CRIg extracellular domain, said complement inhibitory domain, and said synergetic domain in sequence from an N-terminus to a C-terminus, or, which comprises said complement inhibitory domain, said CRIg extracellular domain, and said synergetic domain in sequence from the N-terminus to the C-terminus.

12. The fusion protein according to any one of claims 1 to 11, comprising an amino acid sequence as set forth in SEQ ID NO.: 14.

13. The fusion protein according to any one of claims 1 to 7, wherein said synergetic domain comprises an IgG Fc domain.

14. The fusion protein according to any one of claims 1 to 13, wherein said IgG comprises a protein selected from the group consisting of: human IgG1 and human IgG4.

15. The fusion protein according to any one of claims 1 to 14, wherein said IgG Fc domain comprises an amino acid sequence as set forth in any one of SEQ ID NOs.: 10, 30, 32, and 34.

16. The fusion protein according to any one of claims 13 to 14, comprising a first polypeptide chain and a second polypeptide chain, wherein said first polypeptide chain comprises said CRIg extracellular domain, a first complement inhibitory domain, and a first said IgG Fc domain, and said second polypeptide chain comprises said CRIg extracellular domain, a second complement inhibitory domain, and a second IgG Fc structure domain, and wherein said first IgG Fc domain and said second IgG Fc domain are capable of interacting with each other to form a dimer.

17. The fusion protein according to claim 16, wherein said first complement inhibitory domain and said second complement inhibitory domain each independently comprise a protein selected from the group consisting of factor H (FH), CD55, CD46, CD59 and CR1, or a functional fragment thereof.

18. The fusion protein according to any one of claims 16 to 17, wherein said first complement inhibitory domain is identical to said second complement inhibitory domain.

19. The fusion protein according to claim 18, wherein said first IgG Fc domain is identical to said second IgG Fc domain.

20. The fusion protein according to any one of claims 18 to 19, wherein said first IgG Fc domain and said second IgG Fc domain each comprise an amino acid sequence as set forth in any one of SEQ ID NOs.: 10 and 30.

21. The fusion protein according to any one of claims 18 to 20, wherein said first polypeptide chain is identical to said second polypeptide chain.

22. The fusion protein according to any one of claims 18 to 21, wherein said first polypeptide chain and/or said second polypeptide chain comprise/comprises an amino acid sequence as set forth in any one of SEQ ID NOs.: 14, 24, 26, 28, 58, 60, 66, and 68.

23. The fusion protein according to any one of claims 16 to 17, wherein said first complement inhibitory domain is different from said second complement inhibitory domain.

24. The fusion protein according to claim 23, wherein said first complement inhibitory domain and said second complement inhibitory domain each independently comprise a protein selected from the group consisting of CD59 and CD55, or a functional fragment thereof.

25. The fusion protein according to any one of claims 23 to 24, wherein said first complement inhibitory domain and said second complement inhibitory domain each independently comprise a protein selected from the group consisting of FH and CD55, or a functional fragment thereof.

26. The fusion protein according to any one of claims 23 to 25, wherein said first complement inhibitory domain and said second complement inhibitory domain each independently comprise a protein selected from the group consisting of CD46 and CD59, or a functional fragment thereof.

27. The fusion protein according to any one of claims 23 to 26, wherein said first IgG Fc domain is identical to said second IgG Fc domain.

28. The fusion protein according to any one of claims 23 to 26, wherein said first IgG Fc domain is different from said second IgG Fc domain.

29. The fusion protein according to 28, wherein said first IgG Fc domain comprises an amino acid sequence as set forth in any one of SEQ ID NOs.: 32 and 34.

30. The fusion protein according to any one of claims 28 to 29, wherein said second IgG Fc domain comprises an amino acid sequence as set forth in any one of SEQ ID NOs.: 32 and 34.

31. The fusion protein according to any one of claims 28 to 30, wherein said first polypeptide chain comprises an amino acid sequence as set forth in any one of SEQ ID NOs.: 36, 38, 40, and 42.

32. The fusion protein according to any one of claims 28 to 31, wherein said second polypeptide chain comprises an amino acid sequence as set forth in any one of SEQ ID NOs.: 36, 38, 40, and 42.

33. The fusion protein according to any one of claims 23-32, wherein:
(1) said first polypeptide chain comprises an amino acid sequence as set forth in SEQ ID NO.: 38, and said second polypeptide chain comprises an amino acid sequence as set forth in SEQ ID NO.: 40;
(2) said first polypeptide chain comprises an amino acid sequence as set forth in SEQ ID NO.: 36, and said second polypeptide chain comprises an amino acid sequence as set forth in SEQ ID NO.: 42; or
(3) said first polypeptide chain comprises an amino acid sequence as set forth in SEQ ID NO.: 38, and the second polypeptide chain comprises an amino acid sequence as set forth in SEQ ID NO.: 42.

34. An isolated nucleic acid or isolated nucleic acid molecules, encoding the fusion protein of any one of claims 1 to 33, or a fragment thereof.

35. A vector, comprising the nucleic acid molecule of claim 34.

36. A cell, comprising the vector of claim 35, or expressing the fusion protein of any one of claims 1 to 33.

37. A preparation method of the fusion protein of any one of claims 1 to 33, comprising the steps of: synthesizing the fusion protein of any one of claims 1 to 33, and/or, culturing the cell of claim 36 under a condition of allowing the expression of the fusion protein of any one of claims 1 to 33.

38. A pharmaceutical composition, comprising the fusion protein of any one of claims 1 to 33 and an optionally pharmaceutically acceptable carrier.

39. Use of the fusion protein of any one of claims 1 to 33 or the pharmaceutical composition of claim 37 in the preparation of a drug for treatment of diseases associated with targeted inhibition of complement activation.

40. The use according to any one of claims 39, wherein said diseases comprise autoimmune diseases.

41. The use according to any one of claims 39 to 40, wherein said diseases comprise autoimmune myasthenia gravis.
